# EUROPÄISCHE PATENTSCHRIFT

(11) **EP 2 395 966 B1**
(45) Veröffentlichungstag und Bekanntmachung des Hinweises auf die Patenterteilung: **06.07.2016**
(21) Anmeldenummer: 10703420.9
(22) Anmeldetag: 05.02.2010
(51) Int. Cl.: A61K 8/19, A61K 8/96, A61Q 5/00, A61Q 5/02, A61Q 19/00

(54) **KOSMETISCHE ZUBEREITUNG ZUR HAUTPFLEGE**
COSMETIC COMPOSITION FOR SKIN CARE
PRÉPARATION COSMÉTIQUE POUR LE SOIN DE LA PEAU

(30) Priorität: 12.02.2009 AT 2442009
(43) Veröffentlichungstag der Anmeldung: 21.12.2011
(73) Patentinhaber: Lengheim, Hubert, 2213 Bockfliess (AT)
(72) Erfinder: Lengheim, Hubert, 2213 Bockfliess (AT)
(74) Vertreter: Patentanwälte Puchberger, Berger und Partner
(86) Internationale Anmeldenummer: PCT/EP2010/000742
(87) Internationale Veröffentlichungsnummer: WO 2010/091827

(56) Entgegenhaltungen:
- EP-A1- 0 217 975
- WO-A1-95/13793
- WO-A2-02/092044
- US-A- 5 376 379
- US-A- 5 690 946
- Anonymous: "Natur Therme Templin - Die Thermalsole" 22. April 2008 (2008-04-22), XP002592888 Gefunden im Internet: URL:http://web.archive.org/web/20080422062 638/http://www.naturthermetemplin.de/therm alsole.html [gefunden am 2010-07-20]
- "Wasser und Salz stärken den Wirtschaftsstandort" FÜRSTENFELDER GRENZLANDECHO, Nr. 7, 12. August 2008 (2008-08-12), Seiten 1-24, XP002592889
- Anonym: "Soleangebote in der Spreewald Therme" SPREEWALD THERME XP002592890 Gefunden im Internet: URL:http://www.spreewaldtherme.de/fileadmi n/dam/downloads/06-41.017-7_Fly_Sole.pdf [gefunden am 2010-07-20]
- Anonym: "Bad Wilsnack Eisen- und jodhaltiges Thermal-Sole-Heilwasser" KRISTALLTHERME (Kur- und Gradier-Therme Bad Wilsnack) XP002592891 Gefunden im Internet: URL:http://www.kristalltherme-bad-wilsnack .de/Download_Documents/BW_Thermal_Sole_Hei lwasser.pdf [gefunden am 2010-07-20]
- "International Cosmetic Ingredient Dictionary and Handbook", 2008, The Cosmetic, Toiletry, and Fragrance Association vol. 1

## Beschreibung

Die vorliegende Erfindung betrifft eine kosmetische Zubereitung zur Hautpflege.

Hautkrankheiten wie beispielsweise Neurodermitis und Psoriasis müssen von erfahrenen Dermatologen diagnostiziert und behandelt werden. Trockene, gerötete Haut, Juckreiz sowie schubweises Auftreten von Entzündungen sind häufig Begleiterscheinung von Hauterkrankungen, die die Symptomatik des atopischen Hautbildes zeigen. Sehr oft muss bei akuten Schüben mit einer Cortisonbehandlung begonnen werden.

Neben der ärztlichen Behandlung ist es besonders wichtig, dem Patienten ein gezieltes, insbesondere cortisonfreies, Hautpflegesystem mit Wirkstoffen für die tägliche Pflege zur Verfügung zu stellen, das selbst bei empfindlichen und geröteten Stellen angewandt werden kann. Diese Produkte müssen folgende Eigenschaften aufweisen:
- Hautrötungen entgegenwirken,
- Hautreizungen lindern,
- Entzündungsreaktionen mindern,
- Juckreiz vermindern,
- freie Radikale egalisieren,
- Hautschutzbarriereschicht verstärken,
- a Hautfeuchtigkeit regulieren und
- ausreichende Rückfettung gewährleisten.

Aus US 5 690 946 A ist die Verwendung eines Thermalwassers und/oder Mineralwassers mit einer Mineralisierung von mindestens 700 mg/l zur Linderung der hautirritierenden Wirkung des keratolytischen Inhaltsstoffs eines Kosmetikums zur Behandlung von Akne oder Falten bekannt.

Aus US 5 376 379 ist die die Stabilisierung von Thermalwasser aufweisenden Liposomen durch ein DNA-Gel bekannt.

WO 95/13793 beschreibt eine Zusammensetzung in Gel-Form, die 99 bis 99,9 Gew.-% Thermalwasser, Carboxypolymethylen und ein Neutralisationsmittel aufweist.

Die vorliegende Erfindung gibt eine kosmetische Zubereitung zur Hautpflege gemäss Anspruch 1 an. Diese kosmetische Zubereitung ist erfindungsgemäß dadurch gekennzeichnet, dass sie, bezogen auf die gesamte Zubereitung, neben herkömmlichen kosmetischen Hilfs- und Zusatzstoffen mindestens 2% einer hydrophilen Komponente, mindestens 10% Natrium-Chlorid-Eisen-Iod-Thermalwasser mit einer Elektrolytsumme von mindestens 25 g/kg, bevorzugt mindestens 30 g/kg, und einem Eisengehalt von mindestens 9 mg/kg sowie gegebenenfalls bis zu 15% einer lipophilen Komponente aufweist.

Gemäß einem weiteren Merkmal der Erfindung beträgt die Elektrolytsumme des Natrium-Chlorid-Eisen-Iod-Thermalwassers mindestens 37 g/kg.

Das Natrium-Chlorid-Eisen-Iod-Thermalwasser ist in der erfindungsgemäßen Zubereitung vorzugsweise in einer Menge von 10 bis 80 Gew.-%, bezogen auf das Gesamtgewicht der Zubereitung, enthalten.

Das Natrium-Chlorid-Eisen-Iod-Thermalwasser ist vorzugsweise ein natürlich vorkommendes Thermalwasser.

Erfindungsgemäß enthält die kosmetische Zubereitung eine hydrophile Komponente, die Aqua (CAS-Nr. 7732-18-5), Glycoproteins (CAS-Nr. 84604-16-0), Panthenol (CAS-Nr. 81-13-0), Propylene Glycol (CAS-Nr. 57-55-6), Olea Europaea Leaf Extract (CAS-Nr. 84012-27-1), Aspalathus Linearis Extract (CAS-Nr. 776295-36-4), Centella Asiatica Extract (CAS-Nr. 84696-21-9), Hamamelis Virginiana Extract (CAS-Nr. 84696-19-5), Nelumbo Nucifera Extract (CAS-Nr. 85085-51-4), Stevia Rebaudiana Extract (CAS-Nr. 91722-21-3) und Hyaluronic Acid (CAS-Nr. 9004-61-9) enthält.

Erfindungsgemäß enthält die hydrophile Komponente, bezogen auf das Gewicht der Komponente, 25-50% Aqua (CAS-Nr. 7732-18-5), 10-25% Glycoproteins (CAS-Nr. 84604-16-0), 10-25% Panthenol (CAS-Nr. 81-13-0), 5-10% Propylene Glycol (CAS-Nr. 57-55-6), 1-5% Olea Europaea Leaf Extract (CAS-Nr. 84012-27-1), 1-5% Aspalathus Linearis Extract (CAS-Nr. 776295-36-4), 1-5% Centella Asiatica Extract (CAS-Nr. 84696-21-9), 1-5% Hamamelis Virginiana Extract (CAS-Nr. 84696-19-5), 1-5% Nelumbo Nucifera Extract (CAS-Nr. 85085-51-4), 1-5% Stevia Rebaudiana Extract (CAS-Nr. 91722-21-3) und <0,1% Hyaluronic Acid (CAS-Nr. 9004-61-9).

Gemäß einer speziellen Ausführungsform der Erfindung hat die hydrophile Komponente die in der nachstehenden Tabelle 1 angegebene Zusammensetzung:

**Tabelle 1: Zusammensetzung einer bevorzugten Ausführungsform der hydrophilen Komponente gemäß der Erfindung**

| **Inhaltsstoff nach INCI** | **EINECS**-**Nummer** | **CAS-Nummer** | **Anteil in %** |
|---|---|---|---|
| AQUA | 231-791-2 | 7732-18-5 | 48,9400 |
| GLYCOPROTEINS | 283-294-5 | 84604-16-0 | 20,0000 |
| PANTHENOL | 2013273 | 81-13-0 | 10,0000 |
| PROPYLENE GLYCOL | 2003380 | 57-55-6 | 6,0000 |
| OLEA EUROPAEA LEAF EXTRACT | 310-127-6 | 84012-27-1 | 3,0000 |
| ASPALATHUS LINEARIS EXTRACT | | 776295-36-4 | 3,0000 |
| CENTELLA ASIATICA EXTRACT | 283-640-5 | 84696-21-9 | 3,0000 |
| HAMAMELIS VIRGINIANA EXTRACT | 283-637-9 | 84696-19-5 | 2,2500 |
| NELUMBO NUCIFERA EXTRACT | 2853792 | 85085-51-4 | 2,2500 |
| STEVIA REBAUDIANA EXTRACT | 294-422-4 | 91722-21-3 | 1,5000 |
| HYALURONIC ACID | 2326780 | 9004-61-9 | 0,0600 |

Die hydrophile Komponente ist in der kosmetischen Zubereitung vorzugsweise in einer Menge von 2 bis 10 Gew.-%, bezogen auf das Gesamtgewicht der Zubereitung, enthalten.

Erfindungsgemäß kann die kosmetische Zubereitung eine lipophile Komponente enthalten. Gemäß einem Merkmal enthält die lipophile Komponente einen oder mehrere Inhaltsstoffe, die aus der aus Macadamia Ternifolia Seed Oil (CAS-Nr. 128497-20-1/129811-19-4), Simmondsia Chinensis Seed Oil (CAS-Nr. 61789-91-1), Butyrospermum Parkii Butter (CAS-Nr. 91080-23-8), Panthenyl Triacetate (CAS-Nr. 94089-18-6), Farnesyl Acetate (CAS-Nr. 29548-30-9), Farnesol (CAS-Nr. 4602-84-0), Zea Mays Germ Oil (CAS-Nr. 8001-30-7), Argania Spinosa Kernel Oil (CAS-Nr. 223747-87-3), Triticum Vulgare Germ Oil (CAS-Nr. 68917-73-7), Hordeum Vulgare Cera (CAS-Nr. 85251-64-5), Tocopherol (CAS-Nr. 10191-41-0) und Retinyl Palmitate (CAS-Nr. 79-81-2) bestehenden Gruppe ausgewählt sind.

Gemäß einem Merkmal der Erfindung weist die lipophile Komponente, bezogen auf das Gewicht der Komponente, 10-25% Macadamia Ternifolia Seed Oil (CAS-Nr. 128497-20-1/129811-19-4), 10-25% Simmondsia Chinensis Seed Oil (CAS-Nr. 61789-91-1), 10-25% Butyrospermum Parkii Butter (CAS-Nr. 91080-23-8), 10-25% Panthenyl Triacetate (CAS-Nr. 94089-18-6), 10-25% Farnesyl Acetate (CAS-Nr. 29548-30-9), 10-25% Farnesol (CAS-Nr. 4602-84-0), 5-10% Zea Mays Germ Oil (CAS-Nr. 8001-30-7), 5-10% Argania Spinosa Kernel Oil (CAS-Nr. 223747-87-3), 1-5% Triticum Vulgare Germ Oil (CAS-Nr. 68917-73-7), 1-5% Hordeum Vulgare Cera (CAS-Nr. 85251-64-5), < 0,1% Tocopherol (CAS-Nr. 10191-41-0) und < 0,1% Retinyl Palmitate (CAS-Nr. 79-81-2) auf.

Gemäß einer speziellen Ausführungsform der Erfindung hat die lipophile Komponente die in der nachstehenden Tabelle 2 angegebene Zusammensetzung:

**Tabelle 2 : Zusammensetzung einer bevorzugten Ausführungsform der lipophilen Komponente gemäß der Erfindung**

| **Inhaltsstoff nach INCI** | **EINECS-Nummer** | **CAS-Nummer** | **Anteil in %** |
|---|---|---|---|
| MACADAMIA TERNIFOLIA SEED OIL | 273-313-5 | 128497-20-1 / 129811-19-4 | 21,0000 |
| SIMMONDSIA CHINENSIS SEED OIL | 289-964-3 | 61789-91-1 | 15,0000 |
| BUTYROSPERMUM PARKII BUTTER | 293-515-7 | 91080-23-8 | 13,0000 |
| PANTHENYL TRIACETATE | 3021180 | 94089-18-6 | 10,5400 |
| FARNESYL ACETATE | 2496891 | 29548-30-9 | 10,2300 |
| FARNESOL | 2250041 | 4602-84-0 | 10,2300 |
| ZEA MAYS GERM OIL | 232-281-2 | 8001-30-7 | 9,0955 |
| ARGANIA SPINOSA KERNEL OIL | | 223747-87-3 | 5,0000 |
| TRITICUM VULGARE GERM OIL | | 68917-73-7 | 3,8870 |
| HORDEUM VULGARE CERA | 2864762 | 85251-64-5 | 2,0000 |
| TOCOPHEROL | 233-466-0 | 10191-41-0 | 0,0130 |
| RETINYL PALMITATE | 2012285 | 79-81-2 | 0,0046 |

Die lipophile Komponente ist in der erfindungsgemäßen kosmetischen Zubereitung in einer Menge bis zu 15%, bezogen auf das Gesamtgewicht der Zubereitung, enthalten.

In der vorliegenden Beschreibung sowie in den Ansprüchen werden zur eindeutigen Bezeichnung der Inhaltsstoffe der erfindungsgemäßen kosmetischen Zubereitungen die INCI*(lnternational Nomenclature of Cosmetic Ingredients*)-Bezeichnungen und die EINECS(*European Inventory of Existing Commercial Chemical Substances*)-Nummern bzw. die CAS-Nummern angeführt. Ferner erfolgt die Angabe der Konzentrationsbereiche für die in den kosmetischen Zubereitungen enthaltenen Inhaltsstoffe anhand der FDA-Codes. Dabei bedeutet: A ≥ 50%, B = 25-50%, C = 10-25%, D = 5-10%, E = 1-5%, F = 0,1-1%, G < 0,1%.

Erfindungsgemäß kann die kosmetische Zubereitung einen oder mehrere Zusatzstoffe enthalten. Dieser Zusatzstoff bzw. diese Zusatzstoffe ist/sind vorzugsweise ausgewählt aus der Gruppe bestehend aus Allantoin (CAS-Nr. 97-59-6), Adenosintriphosphat (CAS-Nr. 56-65-5), Betula Alba Leaf Extract (CAS-Nr. 84012-15-7), Biotin (CAS-Nr. 58-85-5), Citrus Grandis Oil (CAS-Nr. 8016-20-4), Cinchona Extract (CAS-Nr. 84776-28-3), Camelia Sinensis Extract (CAS-Nr. 84650-60-2), Chamomilla Recutita Extract (CAS-Nr. 84082-60-0), Equisetum Arvense Extract (CAS-Nr. 71011-23-9), Humulus Lupulus Extract (CAS-Nr. 8060-28-4), Niacinamid (CAS-Nr. 98-92-0), Niacin (CAS-Nr. 59-67-6), Panicum Miliaceum Extract (CAS-Nr. 90082-36-3), Salvia Officinalis Extract (CAS-Nr. 84082-79-1), Tilia Cordata Flower Extract (CAS-Nr. 84929-52-2), Urtica Dioica Extract (CAS-Nr. 84012-40-8) und Vanilla Planifolia Extract (CAS-Nr. 84650-63-5). Selbstverständlich können auch andere, üblicherweise in kosmetischen Zubereitungen zur Hautpflege eingesetzte Zusatzstoffe enthalten sein, wie beispielsweise andere Pflanzenextrakte, Pflanzenpresssäfte, Pflanzenöle, Stutenmilch, Ziegenmilch, Eselsmilch etc.

Unter dem Ausdruck Sales, wie er in der vorliegenden Erfindung verwendet wird, werden anorganische Salze aus Mineralwässern verstanden. Sie haben hautpflegende Wirkung.

Die erfindungsgemäße kosmetische Zubereitung enthält auch einen oder mehrere herkömmliche Hilfsstoffe, die aus der aus Emulgatoren, Co-Emulgatoren, Lösungsmitteln, Lösungsvermittlern, Konservierungsmitteln, Antioxidantien, Komplexbildnern, Feuchthaltemitteln, Filmbildnern, Gelbildnern, konsistenzregulierenden Mitteln, Puffern, Stabilisatoren, Antistatika, Lichtschutzfiltern, Färbemitteln, Desodorantien, Duftstoffen, Maskierungsmitteln, Tensiden, schaumregulierenden Mitteln und schaumstabilisierenden Mitteln bestehenden Gruppe ausgewählt sind.

Als Beispiele für Emulgatoren bzw. Co-Emulgatoren, die in einer erfindungsgemäßen kosmetischen Zubereitung enthalten sein können, kann man Steareth-10 (CAS-Nr. 9005-00-9), PEG-12 Glyceryl Laurate (CAS-Nr. 59070-56-3), Laureth-13 (CAS-Nr. 9002-92-0), PEG-36 Castor Oil (CAS-Nr. 61791-12-6), PEG-10 Olive Glycerides, Laureth-13 (CAS-Nr. 9002-92-0) und dergleichen nennen.

Als Beispiele für Tenside, die in einer erfindungsgemäßen kosmetischen Zubereitung enthalten sein können, kann man Polysorbate 85 (CAS-Nr. 9005-70-3), Mipa-Laureth Sulfate (CAS-Nr. 83016-76-6/9062-04-8), Mipa-Lauryl Sulfate (CAS-Nr. 21142-28-9), Disodium Laureth Sulfosuccinate (CAS-Nr. 39354-45-5), Disodium Cocoamphodiacetate (CAS-Nr. 68650-39-5), PEG-36 Castor Oil (CAS-Nr. 61791-12-6), Cocoamidopropyl Betain (CAS-Nr. 263-058-8) und dergleichen nennen.

Als Beispiele für Konservierungsmittel, die in einer erfindungsgemäßen kosmetischen Zubereitung enthalten sein können, kann man Phenoxyethanol (CAS-Nr. 122-99-6), Imidazolidinyl Urea (CAS-Nr. 39236-46-9), Methylparaben (CAS-Nr. 99-76-3), Ethylparaben (CAS-Nr. 120-47-8), Propylparaben (CAS-Nr. 94-13-3), Butylparaben (CAS-Nr. 94-26-8), Isobutylparaben (CAS-Nr. 857259) und dergleichen nennen.

Einer erfindungsgemäßer kosmetischer Zubereitung können außerdem Duftstoffe, Maskierungsmittel bzw. Desodoratien beispielweise in Form geringer Mengen Parfum zugesetzt werden. Als Beispiele für Duftstoffe, Maskierungsmittel bzw. Desodoratien kann man Linalool (CAS-Nr. 78-70-6), Hydroxycitronellal (CAS-Nr. 107-75-5), Hydroxy Methylpentyl-3-cyclohexene-carboxaldehyde (CAS-Nr. 31906-04-4), Citral (CAS-Nr. 5392-40-5), Cinnamyl Alcohol (CAS-Nr. 104-54-1), Amyl Cinnamal (CAS-Nr. 122-40-7), Limonene (EINECS-Nr. 5989-27-5), Hexyl Cinnamal (CAS-Nr. 101-86-0), Citronellol (CAS-Nr. 106-22-9), Elettaria Cardamon Oil (CAS-Nr. 8000-66-6) und dergleichen nennen.

Als Beispiele für Antioxidantien, die in einer erfindungsgemäßen kosmetischen Zubereitung enthalten sein können, kann man Tocopherol (CAS-Nr. 10191-41-0), Salvia Officinalis Extract (CAS-Nr. 84082-79-1), Olea Europaea Leaf Extract (CAS-Nr. 84012-27-1) und dergleichen nennen.

Als Beispiele für konsistenzregulierenden Mittel und schaumregulierende bzw. schaumstabilisierende Mittel, die in einer erfindungsgemäßen kosmetischen Zubereitung enthalten sein können, kann man Cetearyl Alcohol (CAS-Nr. 67762-27-0/8005-44-5), Dimethicone (CAS-Nr. 9006-65-9/63148-62-9), Linoleamide DEA (CAS-Nr. 56863-02-6) und dergleichen nennen.

Als Beispiele für Filmbildner, die in einer erfindungsgemäßen kosmetischen Zubereitung enthalten sein können, kann man Polyquaternium-10 (CAS-Nr. 81859-24-7/53568-66-4), Hyaluronic Acid (CAS-Nr. 9004-61-9) und dergleichen nennen.

Die hydrophile Komponente gemäß der vorliegenden Erfindung ist ein pflegendes Wirkstoffsystem aus Pflanzenextrakten aus Olivenblatt, Rooibosblatt, Tigerkraut, Hamamelis, Lotosblatt und Steviablatt, das zusätzlich Panthenol und Hyaluronsäure sowie ein System verschiedener Oligopeptide aus sorgfältig selektierten Hefestämmen *(Saccharomyces cerevisiae)* aufweist. Diese Komponente kombiniert synergistisch die Eigenschaften der einzelnen Bestandteile, insbesondere Radikalfängereigenschaften, entzündungshemmende Wirkungen, positiven Einfluss auf die Zellatmung/Zellerneuerung, immunstimulierende Wirkung,

Bildung eines dünnen viskoelastischen Schutzfilmes auf der Haut, Unterstützung der Schutzbarriere der Haut, spürbare Verbesserung des Hautzustandes sowie Erhöhung der Hautfeuchtigkeit. Die Wirkung der synergistischen hydrophilen Komponente wird durch das erfindungsgemäß verwendete Natrium-Chlorid-Eisen-Iod-Thermalwasser wesentlich verstärkt.

Die lipophile Komponente gemäß der vorliegenden Erfindung enthält pflanzliche Öle, wie Macadamianuss-, Jojoba- und Arganöl, Sheabutter, Panthenylacetat, natürliche Tocopherole, Retinylpalmitat, Farnesol, Farnesylacetat sowie einen Spezialextrakt aus der Gerste. Sie ist reich an ungesättigten essentiellen Fettsäuren (z.B. Linolsäure und andere Omega-Fettsäuren) und deren Estern. Sie ist eine bioaktive Komponente, die zur Hautelastizitätsverbesserung, Hautfeuchtigkeitserhöhung, Hautfettregulierung, Stillung von Juckreiz, Verringerung allergischer Reaktionen (durch Beeinflussung der Histaminfreisetzung) sowie zur Regeneration der epidermalen Barriere beiträgt. Die synergistische Wirkung der Inhaltsstoffe der lipophilen Komponente wird durch das erfindungsgemäß verwendete Natrium-Chlorid-Eisen-Iod-Thermalwasser wesentlich verstärkt.

Die erfindungsgemäße kosmetische Zubereitung ist daher in besonderem Maße geeignet, Hautrötungen entgegenzuwirken, Hautreizungen zu lindern, Entzündungsreaktionen zu mindern, Juckreiz zu vermindern, freie Radikale zu egalisieren, die Hautschutzbarriereschicht zu verstärken, die Hautfeuchtigkeit zu regulieren und ausreichende Rückfettung zu gewährleisten.

Die kosmetischen Zubereitungen eignen sich besonders zur Hautpflege bei Atopikern. Beispielsweise wird der ausgeprägte Juckreiz besser gestillt als mit herkömmlichen Hautpflegeprodukten. Die Wirkung hält auch länger an.

Erfindungsgemäß kann die Zubereitung zur Hautpflege beispielsweise als Creme, Lotion, Haartonikum oder Dusch-Shampoo hergestellt werden.

Die in der kosmetischen Zubereitung enthaltene Menge der lipophilen Komponente hängt von der Form der Zubereitung ab. So können Cremes oder Lotionen - bezogen auf das Gesamtgewicht der Zubereitung - bis zu 15 Gew.-% lipophile Komponente enthalten, Vorzugsweise enthält eine solche Zubereitung 2 bis 15% lipophile Komponente, bezogen auf das Gesamtgewicht der Zubereitung. Die lipophile Komponente ist vorzugsweise die lipophile Komponente gemäß Tabelle 2. Demgegenüber enthält ein Haartonikum, Dusch-Shampoo oder dergleichen geringere Mengen an Inhaltsstoffen, die der lipophilen Komponente zuzuordnen sind. Vorzugsweise sind diese Inhaltsstoffe aus Panthenyl Triacetate, Farnesyl Acetate, Farnesol und deren Mischungen ausgewählt.

Für eine als Creme hergestellte erfindungsgemäße kosmetische Zubereitung kann folgende Rahmenrezeptur (Tabelle 3) angegeben werden, wobei die FDA-Codes wie oben definiert sind:

**Tabelle 3: Rahmenrezeptur einer Hautcreme gemäß der Erfindung**

| **Inhaltsstoff nach INCI** | **EINECS-Nummer** | **CAS-Nummer** | **FDA-Code** |
|---|---|---|---|
| AQUA | 231-791-2 | 7732-18-5 | A |
| STEARETH-10 | POLYMER | 9005-00-9 | D |
| MYRISTYL MYRISTATE | 221-787-9 | 3234-85-3 | E |
| CETEARYL ISONONANOATE | 284-424-3 | 84878-33-1 | E |
| GLYCERYLSTEARATE | 2507054 | 31566-31-1 | E |
| CETEARYL ALCOHOL | 2670086 | 67762-27-0 8005-44-5 | E |
| GLYCOPROTEINS | 283-294-5 | 84604-16-0 | E |
| MACADAMIA TERNIFOLIA SEED OIL | 273-313-5 | 128497-20-1 | F |
| | | 129811-19-4 | |
| SIMMONDSIA CHINENSIS OIL | | 61789-91-1 | F |
| PANTHENOL | 2013273 | 81-13-0 | F |
| BUTYROSPERMUM PARKII BUTTER | 293-515-7 | 91080-23-8 | F |
| PANTHENYL TRIACETATE | 3021180 | 94089-18-6 | F |
| DIMETHICONE | POLYMER | 9006-65-9 63148-62-9 | F |
| FARNESOL | 2250041 | 4602-84-0 | F |
| FARNESYL ACETATE | 2496891 | 29548-30-9 | F |
| ZEA MAYS GERM OIL | 232-281-2 | 8001-30-7 | F |
| PROPYLENE GLYCOL | 2003380 | 57-55-6 | F |
| PHENOXYETHANOL | 2045897 | 122-99-6 | F |
| IMIDAZOLIDINYL UREA | 254-372-6 | 39236-46-9 | F |
| ARGANIA SPINOSA KERNEL OIL | | 223747-87-3 | F |
| CENTELLA ASIATICA EXTRACT | 283-640-5 | 84696-21-9 | F |
| OLEA EUROPAEA LEAF EXTRACT | 310-127-6 | 84012-27-1 | F |
| ASPALATHUS LINEARIS EXTRACT | n.a. | 776295-36-4 | F |
| TRITICUM VULGARE GERM OIL | | 68917-73-7 | F |
| PARFUM | | | F |
| NELUMBO NUCIFERA EXTRACT | 2853792 | 85085-51-4 | F |
| HAMAMELIS VIRGINIANA EXTRACT | 283-637-9 | 84696-19-5 | F |
| SALES | | | F |
| STEVIA REBAUDIANA EXTRACT | 294-422-4 | 91722-21-3 | F |
| HORDEUM VULGARE CERA | 2864762 | 85251-64-5 | G |
| METHYLPARABEN | 2027857 | 99-76-3 | G |
| ALPHA-ISOMETHYL IONONE | | | G |
| LINALOOL | 2011344 | 78-70-6 | G |
| CITRIC ACID | 2010691 | 77-92-9 | G |
| ETHYLPARABEN | 2043994 | 120-47-8 | G |
| PROPYLPARABEN | 2023077 | 94-13-3 | G |
| BUTYLPARABEN | 2023187 | 94-26-8 | G |
| HYDROXYCITRONELLAL | 2035187 | 107-75-5 | G |
| HYDROXYMETHYLPENTYL 3 CYCLOHEXENECARBOXALDEHYD E | | 31906-04-4 | G |
| HYALURONIC ACID | 2326780 | 9004-61-9 | G |
| CITRAL | 2263946 | 5392-40-5 | G |
| CINNAMYL ALCOHOL | 2032123 | 104-54-1 | G |
| AMYL CINNAMAL | 2045415 | 122-40-7 | G |
| ISOBUTYLPARABEN | 224-208-8 | 857259 | G |
| LIMONENE | 5989-27-5 | | G |
| TOCOPHEROL | 233-466-0 | 10191-41-0 | G |

Für eine als Lotion hergestellte erfindungsgemäße kosmetische Zubereitung kann folgende Rahmenrezeptur (Tabelle 4) angegeben werden, wobei die FDA-Codes wie oben definiert sind:

**Tabelle 4: Rahmenrezeptur einer Lotion gemäß der Erfindung**

| **Inhaltsstoff nach INCI** | **EINECS-Nummer** | **CAS-Nummer** | **FDA-Code** |
|---|---|---|---|
| AQUA | 231-791-2 | 7732-18-5 | A |
| GLYCERIN | 2002895 | 56-81-5 | E |
| ZEA MAYS GERM OIL | 232-281-2 | 8001-30-7 | E |
| CAPRYLIC/CAPRIC TRIGLYCERIDE | 2774522 | 73398-61-5 | E |
| | | 65381-09-1 | |
| POLYSORBATE 85 | POLYMER | 9005-70-3 | F |
| MACADAMIA TERNIFOLIA SEED OIL | 273-313-5 | 128497-20-1 | F |
| | | 129811-19-4 | |
| PROPYLENE GLYCOL | 2003380 | 57-55-6 | F |
| GLYCOPROTEINS | 283-294-5 | 84604-16-0 | F |
| SIMMONDSIA CHINENSIS OIL | | 61789-91-1 | F |
| TRITICUM VULGARE GERM OIL | | 68917-73-7 | F |
| SACCHARIDE ISOMERATE | | 100843-69-4 | F |
| ACRYLATES/ACRYLAMIDE COPOLYMER | POLYMER | | F |
| PETROLATUM | 2323732 | 8009-03-08 | F |
| PANTHENOL | 2013273 | 81-13-0 | F |
| CALENDULA OFFICINALIS EXTRACT | 283-949-5 | 84776-23-8 | F |
| CHAMOMILLA RECUTITA EXTRACT | 282-006-5 | 84082-60-0 | F |
| BUTYROSPERMUM PARKII BUTTER | 293-515-7 | 91080-23-8 | F |
| PANTHENYL TRIACETATE | 302-118-0 | 94089-18-6 | F |
| FARNESYLACETATE | 249-689-1 | 29548-30-9 | F |
| FARNESOL | 225-004-1 | 4602-84-0 | F |
| IMIDAZOLIDINYL UREA | 254-372-6 | 39236-46-9 | F |
| ALLANTOIN | 202-592-8 | 97-59-6 | F |
| CARBOMER | POLYMER | 9007-20-9 | F |
| | | 9003-01-4 | |
| SALES | n.a. | n.a. | F |
| ARGANIA SPINOSA KERNEL OIL | | 223747-87-3 | F |
| OLEA EUROPAEA LEAF EXTRACT | 310-127-6 | 84012-27-1 | F |
| ASPALATHUS LINEARIS EXTRACT | n.a. | 776295-36-4 | F |
| CENTELLA ASIATICA EXTRACT | 283-640-5 | 84696-21-9 | F |
| PARFUM | | | F |
| METHYLPARABEN | 2027857 | 99-76-3 | F |
| NELUMBO NUCIFERA EXTRACT | 2853792 | 85085-51-4 | G |
| HAMAMELIS VIRGINIANA EXTRACT | 283-637-9 | 84696-19-5 | G |
| HORDEUM VULGARE CERA | 2864762 | 85251-64-5 | G |
| STEVIA REBAUDIANA EXTRACT | 294-422-4 | 91722-21-3 | G |
| PROPYLPARABEN | 2023077 | 94-13-3 | G |
| CITRUS GRANDIS OIL | n.a. | 8016-20-4 | G |
| VANILLA PLANIFOLIA EXTRACT | 283-521-8 | 84650-63-5 | G |
| ELETTARIA CARDAMON OIL | | 8000-66-6 | G |
| HEXYL CINNAMAL | | 101-86-0 | G |
| BENZYLBENZOATE | 204-402-9 | 120-51-4 | G |
| LINALOOL | 201-134-4 | 78-70-6 | G |
| LIMONENE | 5989-27-5 | | G |
| HYALURONIC ACID | 2326780 | 9004-61-9 | G |
| ALPHA-ISOMETHYL IONONE | | | G |
| TOCOPHEROL | 233-466-0 | 10191-41-0 | G |
| CITRONELLOL | 203-375-0 | 106-22-9 | G |
| RETINYL PALMITATE | 2012285 | 79-81-2 | G |

Für eine als Haartonikum hergestellte erfindungsgemäße kosmetische Zubereitung kann folgende Rahmenrezeptur (Tabelle 5) angegeben werden, wobei die FDA-Codes wie oben definiert sind:

**Tabelle 5: Rahmenrezeptur eines Haartonikums gemäß der Erfindung**

| **Inhaltsstoff nach INCI** | **EINECS**-**Nummer** | **CAS-Nummer** | **FDA-Code** |
|---|---|---|---|
| AQUA | 231-791-2 | 7732-18-5 | A |
| MIPA-LAURETH SULFATE | POLYMER | 83016-76-6 | C |
| | | 9062-04-8 | |
| MIPA-LAURYL SULFATE | 2442385 | 21142-28-9 | E |
| COCOAMIDOPROPYL BETAIN | 61789-40-4 | 263-058-8 | E |
| DISODIUM LAURETH SULFOSUCCINATE | POLYMER | 39354-45-5 | E |
| PEG-12 GLYCERYL LAURATE | POLYMER | 59070-56-3 | E |
| DISODIUM COCOAMPHODIACETATE | 2720435 | 68650-39-5 | E |
| LAURETH-13 | POLYMER | 9002-92-0 | E |
| GLYCOPROTEINS | 283-294-5 | 84604-16-0 | E |
| PEG-36 CASTOR OIL | POLYMER | 61791-12-6 | F |
| PROPYLENE GLYCOL | 2003380 | 57-55-6 | F |
| BUTYLENE GLYCOL | 2035297 | 107-88-0 | F |
| PANTHENOL | 2013273 | 81-13-0 | F |
| LINOLEAMIDE DEA | 2604102 | 56863-02-6 | F |
| FARNESYL ACETATE | 2496891 | 29548-30-9 | F |
| FARNESOL | 2250041 | 4602-84-0 | F |
| PARFUM | n.a. | n.a. | F |
| PEG-10 OLIVE GLYCERIDES | POLYMER | n.a. | F |
| IMIDAZOLIDINYL UREA | 254-372-6 | 39236-46-9 | F |
| HYDROLYZED MILK PROTEIN | 2965752 | 92797-39-2 | F |
| | | 8049-98-7 | |
| OLEA EUROPAEA LEAF EXTRACT | 310-127-6 | 84012-27-1 | F |
| CENTELLA ASIATICA EXTRACT | 283-640-5 | 84696-21-9 | F |
| ASPALATHUS LINEARIS EXTRACT | n.a. | 776295-36-4 | F |
| SALES | n.a. | n.a. | F |
| NELUMBO NUCIFERA EXTRACT | 2853792 | 85085-51-4 | F |
| HAMAMELIS VIRGINIANA EXTRACT | 283-637-9 | 84696-19-5 | F |
| METHYLPARABEN | 2027857 | 99-76-3 | F |
| PANICUM MILIACEUM EXTRACT | 2901-25-9 | 90082-36-3 | F |
| SALVIA OFFICINALIS EXTRACT | 282-025-9 | 84082-79-1 | F |
| ADENOSINE TRIPHOSPHATE | 2002832 | 56-65-5 | F |
| BETULA ALBA LEAF EXTRACT | 281-660-9 | 84012-15-7 | F |
| POLYQUATERNIUM-10 | POLYMER | 81859-24-7 | F |
| | | 53568-66-4 | |
| CHAMOMILLA RECUTITA EXTRACT | 282-006-5 | 84082-60-0 | F |
| NIACINAMIDE | 2027134 | 98-92-0 | F |
| CAMELIA SINENSIS EXTRACT | 283-519-7 | 84650-60-2 | G |
| TILIA CORDATA FLOWER EXTRACT | 284-536-2 | 84929-52-2 | G |
| URTICA DIOICA EXTRACT | 281-685-5 | 84012-40-8 | G |
| PANTHENYL TRIACETATE | 3021180 | 94089-18-6 | G |
| HUMULUS LUPULUS EXTRACT | 232-504-3 | 8060-28-4 | G |
| CINCHONA PUBESCENS EXTRACT | 2839537 | 84776-28-3 | G |
| EQUISETUM ARVENSE EXTRACT | 275-123-8 | 71011-23-9 | G |
| STEVIA REBAUDIANA EXTRACT | 294-422-4 | 91722-21-3 | G |
| CITRUS GRANDIS OIL | n.a. | 8016-20-4 | G |
| VANILLA PLANIFOLIA EXTRACT | 283-521-8 | 84650-63-5 | G |
| ELETTARIA CARDAMON OIL | | 8000-66-6 | G |
| PROPYLPARABEN | 202-307-7 | 94-13-3 | G |
| HEXYL CINNAMAL | | 101-86-0 | G |
| BENZYLBENZOATE | 2044029 | 120-51-4 | G |
| LINALOOL | 2011344 | 78-70-6 | G |
| LIMONENE | 5989-27-5 | | G |
| NIACIN | 200-441-0 | 59-67-6 | G |
| HYALURONIC ACID | 232-678-0 | 9004-61-9 | G |
| BIOTIN | 200-399-3 | 58-85-5 | G |

Für eine als Dusch-Shampoo hergestellte erfindungsgemäße kosmetische Zubereitung kann folgende Rahmenrezeptur (Tabelle 6) angegeben werden, wobei die FDA-Codes wie oben definiert sind:

**Tabelle 6: Rahmenrezeptur eines Dusch-Shampoos gemäß der Erfindung**

| **Inhaltsstoff nach INCI** | **EINECS-Nummer** | **CAS-Nummer** | **FDA-Code** |
|---|---|---|---|
| AQUA | 231-791-2 | 7732-18-5 | A |
| MIPA-LAURETH SULFATE | POLYMER | 83016-76-6 | C |
| | | 9062-04-8 | |
| MIPA-LAURYL SULFATE | 2442385 | 21142-28-9 | E |
| COCOAMIDOPROPYL BETAIN | 61789-40-4 | 263-058-8 | E |
| DISODIUM LAURETH SULFOSUCCINATE | POLYMER | 39354-45-5 | E |
| LAURETH-13 | POLYMER | 9002-92-0 | E |
| DISODIUM COCOAMPHODIACETATE | 2720435 | 68650-39-5 | E |
| SODIUM CHLORIDE | 2315983 | 7647-14-5 | F |
| PROPYLENE GLYCOL | 2003380 | 57-55-6 | F |
| GLYCOPROTEINS | 283-294-5 | 84604-16-0 | F |
| LINOLEAMIDE DEA | 2604102 | 56863-02-6 | F |
| PANTHENOL | 2013273 | 81-13-0 | F |
| POLYQUATERNIUM-10 | POLYMER | 81859-24-7 | F |
| | | 53568-66-4 | |
| PEG-10 OLIVE GLYCERIDES | POLYMER | | F |
| IMIDAZOLIDINYL UREA | 254-372-6 | 39236-46-9 | F |
| PANICUM MILIACEUM EXTRACT | 2901-25-9 | 90082-36-3 | F |
| CHAMOMILLA RECUTITA EXTRACT | 282-006-5 | 84082-60-0 | F |
| SALVIA OFFICINALIS EXTRACT | 282-025-9 | 84082-79-1 | F |
| BETULA ALBA LEAF EXTRACT | 281-660-9 | 84012-15-7 | F |
| PARFUM | n.a. | n.a. | F |
| TILIA CORDATA FLOWER EXTRACT | 284-536-2 | 84929-52-2 | F |
| URTICA DIOICA EXTRACT | 281-685-5 | 84012-40-8 | F |
| CAMELIA SINENSIS EXTRACT | 283-519-7 | 84650-60-2 | F |
| EQUISETUM ARVENSE EXTRACT | 275-123-8 | 71011-23-9 | F |
| HUMULUS LUPULUS EXTRACT | 232-504-3 | 8060-28-4 | F |
| CINCHONA EXTRACT | 283-953-7 | 84776-28-3 | F |
| SALES | n.a. | n.a. | F |
| PEG-12 GLYCERYL LAURATE | POLYMER | 59070-56-3 | F |
| METHYLPARABEN | 2027857 | 99-76-3 | F |
| PEG-36 CASTOR OIL | POLYMER | 61791-12-6 | G |
| CENTELLA ASIATICA EXTRACT | 283-640-5 | 84696-21-9 | G |
| OLEA EUROPAEA LEAF EXTRACT | 310-127-6 | 84012-27-1 | G |
| ASPALATHUS LINEARIS EXTRACT | n.a. | 776295-36-4 | G |
| HAMAMELIS VIRGINIANA EXTRACT | 283-637-9 | 84696-19-5 | G |
| NELUMBO NUCIFERA EXTRACT | 285-379-2 | 85085-51-4 | G |
| FARNESYLACETATE | 249-689-1 | 29548-30-9 | G |
| CITRUS GRANDIS OIL | n.a. | 8016-20-4 | G |
| STEVIA REBAUDIANA EXTRACT | 294-422-4 | 91722-21-3 | G |
| FARNESOL | 2250041 | 4602-84-0 | G |
| VANILLA PLANIFOLIA EXTRACT | 283-521-8 | 84650-63-5 | G |
| ELETTARIA CARDAMON OIL | | 8000-66-6 | G |
| PROPYLPARABEN | 202-307-7 | 94-13-3 | G |
| NIACIN | 200-441-0 | 59-67-6 | G |
| HEXYL CINNAMAL | | 101-86-0 | G |
| BENZYL BENZOATE | 2044029 | 120-51-4 | G |
| LINALOOL | 2011344 | 78-70-6 | G |
| HYALURONIC ACID | 232-678-0 | 9004-61-9 | G |

Im Folgenden wird die Erfindung an nicht einschränkenden Beispielen erläutert. Alle Angaben sind, sofern nicht anders angegeben, auf das Gewicht bezogen.

### BEISPIELE

### Beispiel 1: Rezeptur und Herstellung einer erfindungsgemäßen Creme

Die nachstehende Tabelle 7 zeigt eine Rezeptur einer erfindungsgemäßen Hautcreme. Diese Creme enthät etwa 73,51% Wasser (zum Teil aus dem Thermalwasser stammend) und die in der nachstehenden Tabelle 7 angegebenen Inhaltsstoffe.

**Tabelle 7: Erfindungsgemäße Hautcreme**

| **Inhaltsstoffe nach INCI** | **EINECS-Nummer** | **CAS-Nummer** | **Anteil in %** |
|---|---|---|---|
| STEARETH-10 | POLYMER | 9005-00-9 | 6,019000 |
| MYRISTYL MYRISTATE | 221-787-9 | 3234-85-3 | 4,000000 |
| CETEARYL ISONONANOATE | 284-424-3 | 84878-33-1 | 2,990000 |
| GLYCERYL STEARATE | 2507054 | 31566-31-1 | 1,989000 |
| CETEARYL ALCOHOL | 2670086 | 67762-27-0 | 1,521000 |
| | | 8005-44-5 | |
| GLYCOPROTEINS | 283-294-5 | 84604-16-0 | 1,400000 |
| MACADAMIA TERNIFOLIA SEED OIL | 273-313-5 | 128497-20-1 | 0,987000 |
| | | 129811-19-4 | |
| SIMMONDSIA CHINENSIS OIL | | 61789-91-1 | 0,705000 |
| PANTHENOL | 2013273 | 81-13-0 | 0,700000 |
| BUTYROSPERMUM PARKII BUTTER | 293-515-7 | 91080-23-8 | 0,611000 |
| PANTHENYL TRIACETATE | 3021180 | 94089-18-6 | 0,495380 |
| DIMETHICONE | POLYMER | 9006-65-9 | 0,481000 |
| | | 63148-62-9 | |
| FARNESOL | 2250041 | 4602-84-0 | 0,480823 |
| FARNESYL ACETATE | 2496891 | 29548-30-9 | 0,480810 |
| ZEA MAYS GERM OIL | 232-281-2 | 8001-30-7 | 0,427486 |
| PROPYLENE GLYCOL | 2003380 | 57-55-6 | 0,420000 |
| PHENOXYETHANOL | 2045897 | 122-99-6 | 0,370000 |
| IMIDAZOLIDINYL UREA | 254-372-6 | 39236-46-9 | 0,300000 |
| ARGANIA SPINOSA KERNEL OIL | | 223747-87-3 | 0,235000 |
| CENTELLA ASIATICA EXTRACT | 283-640-5 | 84696-21-9 | 0,210000 |
| OLEA EUROPAEA LEAF EXTRACT | 310-127-6 | 84012-27-1 | 0,210000 |
| ASPALATHUS LINEARIS EXTRACT | n.a. | 776295-36-4 | 0,210000 |
| TRITICUM VULGARE GERM OIL | | 68917-73-7 | 0,182689 |
| PARFUM | | | 0,176213 |
| NELUMBO NUCIFERA EXTRACT | 2853792 | 85085-51-4 | 0,157500 |
| HAMAMELIS VIRGINIANA EXTRACT | 283-637-9 | 84696-19-5 | 0,157500 |
| SALES | | | 0,150000 |
| STEVIA REBAUDIANA EXTRACT | 294-422-4 | 91722-21-3 | 0,105000 |
| HORDEUM VULGARE CERA | 2864762 | 85251-64-5 | 0,094000 |
| METHYLPARABEN | 2027857 | 99-76-3 | 0,085000 |
| ALPHA-ISOMETHYL IONONE | | | 0,025575 |
| LINALOOL | 2011344 | 78-70-6 | 0,023500 |
| CITRIC ACID | 2010691 | 77-92-9 | 0,020000 |
| ETHYLPARABEN | 2043994 | 120-47-8 | 0,020000 |
| PROPYLPARABEN | 2023077 | 94-13-3 | 0,015000 |
| BUTYLPARABEN | 2023187 | 94-26-8 | 0,007500 |
| HYDROXYCITRONELLAL | 2035187 | 107-75-5 | 0,006825 |
| HYDROXYMETHYLPENTYL 3 CYCLOHEXENECARBOXALDEHYD E | | 31906-04-4 | 0,004500 |
| HYALURONIC ACID | 2326780 | 9004-61-9 | 0,004200 |
| CITRAL | 2263946 | 5392-40-5 | 0,004075 |
| CINNAMYL ALCOHOL | 2032123 | 104-54-1 | 0,003000 |
| AMYL CINNAMAL | 2045415 | 122-40-7 | 0,002875 |
| ISOBUTYLPARABEN | 224-208-8 | 857259 | 0,002500 |
| LIMONENE | 5989-27-5 | | 0,001750 |
| TOCOPHEROL | 233-466-0 | 10191-41-0 | 0,000611 |

Die obige Hautcreme ist eine leichte, schnell einziehende Creme, die neben Hilfs- und Zusatzstoffen 4,7% lipophile Komponente, die aus Macadamia Ternifolia Seed Oil, Simmondsia Chinensis Seed Oil, Butyrospermum Parkii Butter, Panthenyl Triacetate, Farnesyl Acetate, Farnesol, Zea Mays Germ Oil, Argania Spinosa Kernel Oil, Triticum Vulgare Germ Oil, Hordeum Vulgare Cera und Tocopherol besteht, 7% hydrophile Komponente gemäß Tabelle 1 sowie 30% Natrium-Chlorid-Eisen-Iod-Thermalwasser enthält.

Die erfindungsgemäße Hautcreme kann in folgenden Schritten hergestellt werden:

### Definitionen:

Phase A: Fettphase (öl- und fettlösliche Bestandteile) einschließlich einer Mischung von Emulgatoren und Konsistenzgebern, die Steareth-10, Cetearyl Isononanoate, Glyceryl Stearate, Cetearyl Alcohol und Dimethicone enthält, und der lipophilen Komponente
Phase B: Wasserphase (Wasser, Natrium-Chlorid-Eisen-Iod-Thermalwasser, hydrophile Komponente und andere wasserlösliche Bestandteile)
Phase C: Parfumöl, wärmeempfindliche Wirkstoffe

Die zur Herstellung der Creme verwendete Anlage beinhaltet einen beheizbaren Doppelmantelkessel mit Rührwerk. Vorzugsweise ist auch ein Homogenisator vorgesehen sowie die Möglichkeit, Vakuum anzulegen.
1.) Die Phase A wird im Rührkessel vorgelegt, der Rührkessel unter Vakuum gesetzt und auf 75°C erhitzt (Wassermanteltemperatur ca. 80°C), ab ca. 50°C wird das Rührwerk eingeschaltet (20-30 U/min.)
2.) Das eingesetzte Wasser und Thermalwasser der Phase B wird vor der Verwendung durch eine Sterilkerze filtriert.
3.) Feste Bestandteile der Phase B werden in einer geeigneten Teilmenge Wasser vorgelöst, Allantoin wird während des Aufheizens unter Rühren gelöst. Die Phase B wird vereinigt.
4.) Die Phase B wird auf Herdplatten auf 75°C aufgeheizt.
5.) Ein Teil des Wassers (Phase B) wird entnommen, um das Konservierungsmittel Imidazolidinyl Urea zu lösen. Diese Lösung wird später während der Abkühlphase (siehe Punkt 9.) zugeführt.
6.) Haben die beiden Phasen 75°C erreicht, wird die Phase A für 60 Sekunden homogenisiert, die Rührgeschwindigkeit auf 55 U/min erhöht. Anschließend wird die Phase B in das Rührwerk über Vakuum eingesaugt und für 90 Sekunden homogenisiert.
7.) Für 30 Minuten wird die Emulsion auf 75°C gehalten und mit 55 U/min unter Vakuum gerührt.
8.) Nach 30 Minuten wird mit dem Kühlen begonnen. Der Wasserdurchfluss beträgt etwa 1,5 l/min, wobei der Unterschied Mantel- zu Produkttemperatur nicht größer als 10°C sein soll.
9.) Bei Erreichen von 55°C Produkttemperatur wird das in Wasser gelöste Imidazolidinyl Urea über den Trichter in das Rührwerk eingesaugt, für 90 Sekunden homogenisiert und weiter abgekühlt.
10.) Bei Erreichen von 35°C Produkttemperatur werden die Bestandteile der Phase C getrennt über den Trichter in das Rührwerk eingesaugt, für 90 Sekunden homogenisiert und weiter abgekühlt.
11.) Bei Erreichen von ca. 28 °C Produkttemperatur wird das Vakuum abgesetzt, das Kühlwasser abgedreht, das Rührwerk langsam weiterlaufen gelassen (ca. 25 U/min.) und das Produkt über den Homogenisator in geeignete Gebinde abgefüllt.

### Beispiel 2: Rezeptur und Herstellung einer erfindungsgemäßen Körperlotion

Die nachstehende Tabelle 8 zeigt eine Rezeptur einer erfindungsgemäßen Körperlotion.

Diese Lotion enthält etwa 84,81% Wasser (zum Teil aus dem Thermalwasser stammend) und die und die in der nachstehenden Tabelle 8 angegebenen Inhaltsstoffe.

**Tabelle 8: Erfindungsgemäße Lotion**

| **Inhaltsstoff nach INCI** | **EINECS**-**Nummer** | **CAS-Nummer** | **Anteil in %** |
|---|---|---|---|
| GLYCERIN | 2002895 | 56-81-5 | 1,70000 |
| ZEA MAYS GERM OIL | 232-281-2 | 8001-30-7 | 1,35232 |
| CAPRYLIC/CAPRIC TRIGLYCERIDE | 2774522 | 73398-61-5 | 1,24196 |
| | | 65381-09-1 | |
| POLYSORBATE 85 | POLYMER | 9005-70-3 | 0,96000 |
| MACADAMIA TERNIFOLIA SEED OIL | 273-313-5 | 128497-20-1 | 0,83000 |
| | | 129811-19-4 | |
| PROPYLENE GLYCOL | 2003380 | 57-55-6 | 0,80000 |
| GLYCOPROTEINS | 283-294-5 | 84604-16-0 | 0,80000 |
| SIMMONDSIA CHINENSIS OIL | | 61789-91-1 | 0,65000 |
| TRITICUM VULGARE GERM OIL | | 68917-73-7 | 0,59277 |
| SACCHARIDE ISOMERATE | | 100843-69-4 | 0,55000 |
| ACRYLATES/ACRYLAMIDE COPOLYMER | POLYMER | | 0,54000 |
| PETROLATUM | 2323732 | 8009-03-08 | 0,50000 |
| PANTHENOL | 2013273 | 81-13-0 | 0,40000 |
| CALENDULA OFFICINALIS EXTRACT | 283-949-5 | 84776-23-8 | 0,40000 |
| CHAMOMILLA RECUTITA EXTRACT | 282-006-5 | 84082-60-0 | 0,40000 |
| BUTYROSPERMUM PARKII BUTTER | 293-515-7 | 91080-23-8 | 0,39000 |
| PANTHENYL TRIACETATE | 302-118-0 | 94089-18-6 | 0,31620 |
| FARNESYL ACETATE | 249-689-1 | 29548-30-9 | 0,30690 |
| FARNESOL | 225-004-1 | 4602-84-0 | 0,30690 |
| IMIDAZOLIDINYL UREA | 254-372-6 | 39236-46-9 | 0,30000 |
| ALLANTOIN | 202-592-8 | 97-59-6 | 0,30000 |
| CARBOMER | POLYMER | 9007-20-9 | 0,25000 |
| | | 9003-01-4 | |
| SALES | n.a. | n.a. | 0,15000 |
| ARGANIA SPINOSA KERNEL OIL | | | 0,15000 |
| OLEA EUROPAEA LEAF EXTRACT | 310-127-6 | 84012-27-1 | 0,12000 |
| ASPALATHUS LINEARIS EXTRACT | n.a. | 776295-36-4 | 0,12000 |
| CENTELLA ASIATICA EXTRACT | 283-640-5 | 84696-21-9 | 0,12000 |
| PARFUM | | | 0,11642 |
| METHYLPARABEN | 2027857 | 99-76-3 | 0,11000 |
| NELUMBO NUCIFERA EXTRACT | 2853792 | 85085-51-4 | 0,09000 |
| HAMAMELIS VIRGINIANA EXTRACT | 283-637-9 | 84696-19-5 | 0,09000 |
| HORDEUM VULGARE CERA | 2864762 | 85251-64-5 | 0,06000 |
| STEVIA REBAUDIANA EXTRACT | 294-422-4 | 91722-21-3 | 0,06000 |
| PROPYLPARABEN | 2023077 | 94-13-3 | 0,03000 |
| CITRUS GRANDIS OIL | n.a. | 8016-20-4 | 0,02000 |
| VANILLA PLANIFOLIA EXTRACT | 283-521-8 | 84650-63-5 | 0,01600 |
| ELETTARIA CARDAMON OIL | | 8000-66-6 | 0,01500 |
| HEXYL CINNAMAL | | 101-86-0 | 0,00860 |
| BENZYLBENZOATE | 204-402-9 | 120-51-4 | 0,00730 |
| LINALOOL | 201-134-4 | 78-70-6 | 0,00600 |
| LIMONENE | 5989-27-5 | | 0,00417 |
| HYALURONIC ACID | 2326780 | 9004-61-9 | 0,00240 |
| ALPHA-ISOMETHYL IONONE | | | 0,00200 |
| TOCOPHEROL | 233-466-0 | 10191-41-0 | 0,00239 |
| CITRONELLOL | 203-375-0 | 106-22-9 | 0,00180 |
| RETINYL PALMITATE | 2012285 | 79-81-2 | 0,00056 |

Die obige Körperlotion ist eine leichte, schnell einziehende Lotion. Sie weist einen geringen Emulgatoranteil auf. Neben herkömmlichen Hilfsstoffen, die u.a. Feuchthaltemittel umfassen, enthält sie 4 % hydrophile Komponente gemäß Tabelle 1, 12% Natrium-Chlorid-Eisen-Iod-Thermalwasser, 3% lipophile Komponente gemäß Tabelle 2 sowie Kamillenöl, Ringelblumenöl und Allantoin als weitere Zusatzstoffe.

Die erfindungsgemäße Körperlotion kann in folgenden Schritten hergestellt werden:

### Definitionen:

Phase A: Fettphase (öl- und fettlösliche Bestandteile) einschließlich eines Mikroemulsionenbildners, der Polysorbate 85, ein Blockcopolymer aus Acrylamiden und Acrylaten sowie eine kleine Menge Paraffin enthält
Phase B: Wasserphase (Wasser und wasserlösliche Bestandteile)
Phase C: Parfumöl und zusätzliche Konservierung

Die zur Herstellung der Lotion verwendete Anlage beinhaltet einen beheizbaren Kessel mit Rührwerk. Vorzugsweise ist auch ein Homogenisator vorgesehen sowie die Möglichkeit, Vakuum anzulegen.
1.) Die Phase B wird im Rührkessel vorgelegt. Die benötigte Wassermenge wird in einem geeigneten Gefäß bereitgestellt. Im Wasser werden feste Bestandteile vorgelöst und kosmetische Wirkstoffe, Farbstoffe etc. eingebracht.
2.) Die Bestandteile der Phase C werden einzeln zur Phase B in den Rührkessel zugegeben.
3.) Dann wird das Rührwerk mit ca. 50 U/min in Betrieb genommen und Vakuum angesetzt. Die Rührzeit beträgt 10 Minuten. In dieser Zeit wird 3-mal der Homogenisator für ca. 30 Sekunden laufen gelassen.
4.) Zwischenzeitlich wird die Phase A in einem geeigneten Gefäß vorgemischt, der Mikroemulsionenbildner wird vor Entnahme aus dem Gebinde aufgerührt.
5.) Das Vakuum wird am Rührwerk ablassen, die Vorrichtung geöffnet und die Phase A direkt zugegeben. Die Vorrichtung wird wieder geschlossen, das Rührwerk in Betrieb genommen und Vakuum angelegt, dann für 60 Sekunden homogenisiert.
6.) Das Rührwerk wird für weitere 20 Minuten auf ca. 50 U/min. laufen gelassen und in dieser Zeit 3-mal der Homogenisator für ca. 30 Sekunden laufen gelassen.
7.) Das Vakuum wird abgesetzt, der Rührer langsam weiterlaufen gelassen (ca. 25 U/min) und das Produkt über den Homogenisator in geeignete Gebinde abgefüllt.

### Beispiel 4: Rezeptur eines erfindungsgemäßen Haartonikums

Die nachstehende Tabelle 9 zeigt eine Rezeptur eines erfindungsgemäßen Haartonikums. Dieses Haartonikum enthält etwa 71,2% Wasser (zum Teil aus dem Thermalwasser stammend) und die in der nachstehenden Tabelle 9 angegebenen Inhaltsstoffe.

**Tabelle 9: Erfindungsgemäßes Haartonikum**

| **Inhaltsstoff nach INCI** | **EINECS-Nummer** | **CAS-Nummer** | **Anteil in %** |
|---|---|---|---|
| MIPA-LAURETH SULFATE | POLYMER | 83016-76-6 | 10,00000 |
| | | 9062-04-8 | |
| MIPA-LAURYL SULFATE | 2442385 | 21142-28-9 | 3,15000 |
| COCOAMIDOPROPYL BETAIN | 61789-40-4 | 263-058-8 | 1,60000 |
| DISODIUM LAURETH SULFOSUCCINATE | POLYMER | 39354-45-5 | 1,50000 |
| PEG-12 GLYCERYL LAURATE | POLYMER | 59070-56-3 | 1,20000 |
| DISODIUM COCOAMPHODIACETATE | 2720435 | 68650-39-5 | 1,20000 |
| LAURETH-13 | POLYMER | 9002-92-0 | 1,20000 |
| GLYCOPROTEINS | 283-294-5 | 84604-16-0 | 1,00000 |
| PEG-36 CASTOR OIL | POLYMER | 61791-12-6 | 0,90000 |
| PROPYLENE GLYCOL | 2003380 | 57-55-6 | 0,86000 |
| BUTYLENE GLYCOL | 2035297 | 107-88-0 | 0,60000 |
| PANTHENOL | 2013273 | 81-13-0 | 0,54990 |
| LINOLEAMIDE DEA | 2604102 | 56863-02-6 | 0,45000 |
| FARNESYL ACETATE | 2496891 | 29548-30-9 | 0,45000 |
| FARNESOL | 2250041 | 4602-84-0 | 0,36000 |
| PARFUM | n.a. | n.a. | 0,34927 |
| PEG-10 OLIVE GLYCERIDES | POLYMER | n.a. | 0,30000 |
| IMIDAZOLIDINYL UREA | 254-372-6 | 39236-46-9 | 0,30000 |
| HYDROLYZED MILK PROTEIN | 2965752 | 92797-39-2 | 0,20000 |
| | | 8049-98-7 | |
| OLEA EUROPAEA LEAF EXTRACT | 310-127-6 | 84012-27-1 | 0,15000 |
| CENTELLA ASIATICA EXTRACT | 283-640-5 | 84696-21-9 | 0,15000 |
| ASPALATHUS LINEARIS EXTRACT | n.a. | 776295-36-4 | 0,15000 |
| SALES | n.a. | n.a. | 0,15000 |
| NELUMBO NUCIFERA EXTRACT | 2853792 | 85085-51-4 | 0,11250 |
| HAMAMELIS VIRGINIANA EXTRACT | 283-637-9 | 84696-19-5 | 0,11250 |
| METHYLPARABEN | 2027857 | 99-76-3 | 0,11000 |
| PANICUM MILIACEUM EXTRACT | 2901-25-9 | 90082-36-3 | 0,10000 |
| SALVIA OFFICINALIS EXTRACT | 282-025-9 | 84082-79-1 | 0,10000 |
| ADENOSINE TRIPHOSPHATE | 2002832 | 56-65-5 | 0,10000 |
| BETULA ALBA LEAF EXTRACT | 281-660-9 | 84012-15-7 | 0,10000 |
| POLYQUATERNIUM-10 | POLYMER | 81859-24-7 | 0,10000 |
| | | 53568-66-4 | |
| CHAMOMILLA RECUTITA EXTRACT | 282-006-5 | 84082-60-0 | 0,10000 |
| NIACINAMIDE | 2027134 | 98-92-0 | 0,10000 |
| CAMELIA SINENSIS EXTRACT | 283-519-7 | 84650-60-2 | 0,09000 |
| TILIA CORDATA FLOWER EXTRACT | 284-536-2 | 84929-52-2 | 0,09000 |
| URTICA DIOICA EXTRACT | 281-685-5 | 84012-40-8 | 0,09000 |
| PANTHENYL TRIACETATE | 3021180 | 94089-18-6 | 0,09000 |
| HUMULUS LUPULUS EXTRACT | 232-504-3 | 8060-28-4 | 0,09000 |
| CINCHONA PUBESCENS EXTRACT | 2839537 | 84776-28-3 | 0,09000 |
| EQUISETUM ARVENSE EXTRACT | 275-123-8 | 71011-23-9 | 0,09000 |
| STEVIA REBAUDIANA EXTRACT | 294-422-4 | 91722-21-3 | 0,07500 |
| CITRUS GRANDIS OIL | n.a. | 8016-20-4 | 0,06000 |
| VANILLA PLANIFOLIA EXTRACT | 283-521-8 | 84650-63-5 | 0,04800 |
| ELETTARIA CARDAMON OIL | | 8000-66-6 | 0,04500 |
| PROPYLPARABEN | 202-307-7 | 94-13-3 | 0,03000 |
| HEXYL CINNAMAL | | 101-86-0 | 0,02580 |
| BENZYLBENZOATE | 2044029 | 120-51-4 | 0,02190 |
| LINALOOL | 2011344 | 78-70-6 | 0,01801 |
| LIMONENE | 5989-27-5 | | 0,01252 |
| NIACIN | 200-441-0 | 59-67-6 | 0,01000 |
| HYALURONIC ACID | 232-678-0 | 9004-61-9 | 0,00300 |
| BIOTIN | 200-399-3 | 58-85-5 | 0,00010 |

Das obige Tonikum für Haar und Kopfhaut ist wegen seines hohen Rückfettungsvermögens für die Kopfhaut besonders für Atopiker geeignet. Es enthält 17% Natrium-Chlorid-Eisen-Iod-Thermalwasser, 5,0% hydrophile Komponente gemäß Tabelle 1, insgesamt 0,9% der Inhaltsstoffe Panthenyl Triacetate, Farnesyl Acetate und Farnesol, die der lipophilen Komponente zuzuordnen sind, sowie weitere, insbesondere Haar und Kopfhaut pflegende und die Haarstruktur verbessernde Bestandteile und kosmetische Hilfsstoffe.

### Beispiel 5: Rezeptur und Herstellung eines erfindungsgemäßen Dusch-Shampoos

Die nachstehende Tabelle 10 zeigt eine Rezeptur eines erfindungsgemäßen Dusch-Shampoos. Dieses Dusch-Shampoo enthält etwa 73,77% Wasser (z. T. aus dem Thermalwasser stammend) und die in der nachstehenden Tabelle 10 angegebenen Inhaltsstoffe.

**Tabelle 10: Erfindungsgemäßes Dusch-Shampoo**

| **Inhaltsstoff nach INCI** | **EINECS-Nummer** | **CAS-Nummer** | **Anteil in %** |
|---|---|---|---|
| MIPA-LAURETH SULFATE | POLYMER | 83016-76-6 | 10,00000 |
| | | 9062-04-8 | |
| MIPA-LAURYL SULFATE | 2442385 | 21142-28-9 | 3,15000 |
| COCOAMIDOPROPYL BETAIN | 61789-40-4 | 263-058-8 | 1,60000 |
| DISODIUM LAURETH SULFOSUCCINATE | POLYMER | 39354-45-5 | 1,50000 |
| LAURETH-13 | POLYMER | 9002-92-0 | 1,20000 |
| DISODIUM COCOAMPHODIACETATE | 2720435 | 68650-39-5 | 1,20000 |
| SODIUM CHLORIDE | 2315983 | 7647-14-5 | 0,90000 |
| PROPYLENE GLYCOL | 2003380 | 57-55-6 | 0,71000 |
| GLYCOPROTEINS | 283-294-5 | 84604-16-0 | 0,50000 |
| LINOLEAMIDE DEA | 2604102 | 56863-02-6 | 0,45000 |
| PANTHENOL | 2013273 | 81-13-0 | 0,37475 |
| POLYQUATERNIUM-10 | POLYMER | 81859-24-7 | 0,30000 |
| | | 53568-66-4 | |
| PEG-10 OLIVE GLYCERIDES | POLYMER | | 0,30000 |
| IMIDAZOLIDINYL UREA | 254-372-6 | 39236-46-9 | 0,30000 |
| PANICUM MILIACEUM EXTRACT | 2901-25-9 | 90082-36-3 | 0,25000 |
| CHAMOMILLA RECUTITA EXTRACT | 282-006-5 | 84082-60-0 | 0,25000 |
| SALVIA OFFICINALIS EXTRACT | 282-025-9 | 84082-79-1 | 0,25000 |
| BETULA ALBA LEAF EXTRACT | 281-660-9 | 84012-15-7 | 0,25000 |
| PARFUM | n.a. | n.a. | 0,23285 |
| TILIA CORDATA FLOWER EXTRACT | 284-536-2 | 84929-52-2 | 0,22500 |
| URTICA DIOICA EXTRACT | 281-685-5 | 84012-40-8 | 0,22500 |
| CAMELIA SINENSIS EXTRACT | 283-519-7 | 84650-60-2 | 0,22500 |
| EQUISETUM ARVENSE EXTRACT | 275-123-8 | 71011-23-9 | 0,22500 |
| HUMULUS LUPULUS EXTRACT | 232-504-3 | 8060-28-4 | 0,22500 |
| CINCHONA EXTRACT | 283-953-7 | 84776-28-3 | 0,22500 |
| SALES | n.a. | n.a. | 0,15000 |
| PEG-12 GLYCERYL LAURATE | POLYMER | 59070-56-3 | 0,12000 |
| METHYLPARABEN | 2027857 | 99-76-3 | 0,11000 |
| PEG-36 CASTOR OIL | POLYMER | 61791-12-6 | 0,09000 |
| CENTELLA ASIATICA EXTRACT | 283-640-5 | 84696-21-9 | 0,07500 |
| OLEA EUROPAEA LEAF EXTRACT | 310-127-6 | 84012-27-1 | 0,07500 |
| ASPALATHUS LINEARIS EXTRACT | n.a. | 776295-36-4 | 0,07500 |
| HAMAMELIS VIRGINIANA EXTRACT | 283-637-9 | 84696-19-5 | 0,05625 |
| NELUMBO NUCIFERA EXTRACT | 285-379-2 | 85085-51-4 | 0,05625 |
| FARNESYL ACETATE | 249-689-1 | 29548-30-9 | 0,04500 |
| CITRUS GRANDIS OIL | n.a. | 8016-20-4 | 0,04000 |
| STEVIA REBAUDIANA EXTRACT | 294-422-4 | 91722-21-3 | 0,03750 |
| FARNESOL | 2250041 | 4602-84-0 | 0,03600 |
| VANILLA PLANIFOLIA EXTRACT | 283-521-8 | 84650-63-5 | 0,03200 |
| ELETTARIA CARDAMON OIL | | 8000-66-6 | 0,03000 |
| PROPYLPARABEN | 202-307-7 | 94-13-3 | 0,03000 |
| NIACIN | 200-441-0 | 59-67-6 | 0,02500 |
| HEXYL CINNAMAL | | 101-86-0 | 0,01720 |
| BENZYLBENZOATE | 2044029 | 120-51-4 | 0,01460 |
| LINALOOL | 2011344 | 78-70-6 | 0,01201 |
| HYALURONIC ACID | 232-678-0 | 9004-61-9 | 0,00150 |

Das obige Shampoo ist besonders milde. Es enthält 24% Natrium-Chlorid-Eisen-Iod-Thermalwasser, 2,5% hydrophile Komponente gemäß Tabelle 1, insgesamt 0,09 % der Inhaltsstoffe Farnesyl Acetate und Farnesol, die der der lipophilen Komponente zuzuordnen sind, sowie weitere das Haar und die Kopfhaut pflegende sowie die Haarstruktur verbessernde Wirkstoffe und herkömmliche kosmetische Hilfsstoffe. Das Dusch-Shampoo ist besonders geeignet für Atopiker, da es ausgezeichnete Rückfettung der Kopfhaut gewährleistet.

Die zur Herstellung des Dusch-Shampoos verwendete Anlage beinhaltet einen Kessel mit Rührwerk.

Bei der Herstellung des Dusch-Shampoos werden die Tenside sowie der lipophilen Komponente zuzuordnende Inhaltsstoffe und weitere lipophile Wirk- und Hilfsstoffe im Rührkessel vorgelegt. Parfumöle und ätherische Öle werden direkt in die Tensidmischung eingerührt. Die Zugabe des Wassers, der hydrophilen Komponente und weiterer wasserlöslicher Bestandteile erfolgt unter Rühren.

Die Herstellung kann in folgenden Schritten erfolgen:
1.) Einwaage der Tenside in den Rührkessel.
2.) Es wird mit einem Langsamrührer oder ganz kurz mit einem Schnellrührer gerührt. Parfumöl oder ätherisches Öl werden eingemischt.
3.) Es wird 15 min. gerührt.
4.) Die eingesetzten Polymere werden einer Teilmenge kalten Wassers zugesetzt, es wird bis höchstens 70°C unter Rühren auf dem Wasserbad oder im Heizrührkessel erwärmt. Die klare Lösung wird wieder auf ca. 30°C abgekühlt.
5.) Wasser, das sterilfiltrierte Thermalwasser, die hydrophile Komponente sowie anderen wasserlösliche Bestandteile werden eingerührt.
6.) Die Polymerlösung oder sonstige verdickende Komponenten werden eingerührt.
7.) Es wird mindestens 1 Stunde weitergerührt.
8.) Abschließend wird in die vorgegeben Gebinde abgefüllt.

## Patentansprüche

1. Kosmetische Zubereitung zur Hautpflege, **dadurch gekennzeichnet, dass** sie, bezogen auf das Gesamtgewicht der Zubereitung, neben herkömmlichen kosmetischen Hilfs- und Zusatzstoffen,
(a) mindestens 2% einer hydrophilen Komponente, die Aqua (CAS-Nr. 7732-18-5), Glycoproteins (CAS-Nr. 84604-16-0), Panthenol (CAS-Nr. 81-13-0), Propylene Glycol (CAS-Nr. 57-55-6), Olea Europaea Leaf Extract (CAS-Nr. 84012-27-1), Aspalathus Linearis Extract (CAS-Nr. 776295-36-4), Centella Asiatica Extract (CAS-Nr. 84696-21-9), Hamamelis Virginiana Extract (CAS-Nr. 84696-19-5), Nelumbo Nucifera Extract (CAS-Nr. 85085-51-4), Stevia Rebaudiana Extract (CAS-Nr. 91722-21-3) und Hyaluronic Acid (CAS-Nr. 9004-61-9) enthält,
(b) mindestens 10% Natrium-Chlorid-Eisen-Iod-Thermalwasser mit einer Elektrolytsumme von mindestens 25 g/kg, bevorzugt mindestens 30 g/kg, und einem Eisengehalt von mindestens 9 mg/kg
sowie gegebenenfalls
(c) bis zu 15% einer lipophilen Komponente, die einen oder mehrere Bestandteile ausgewählt aus der Gruppe bestehend aus Macadamia Ternifolia Seed Oil (CAS-Nr. 128497-20-1/129811-19-4), Simmondsia Chinensis Seed Oil (CAS-Nr. 61789-91-1), Butyrospermum Parkii Butter (CAS-Nr. 91080-23-8), Panthenyl Triacetate (CAS-Nr. 94089-18-6), Farnesyl Acetate (CAS-Nr. 29548-30-9), Farnesol (CAS-Nr. 4602-84-0), Zea Mays Germ Oil (CAS-Nr. 8001-30-7), Argania Spinosa Kernel Oil (CAS-Nr. 223747-87-3), Triticum Vulgare Germ Oil (CAS-Nr. 68917-73-7), Hordeum Vulgare Cera (CAS-Nr. 85251-64-5), Tocopherol (CAS-Nr. 10191-41-0) und Retinyl Palmitate (CAS-Nr. 79-81-2) enthält,
enthält.

2. Zubereitung nach Anspruch 1, **dadurch gekennzeichnet, dass** das Natrium-Chlorid-Eisen-Iod-Thermalwasser eine Elektrolytsumme von mindestens 37 g/kg enthält.

3. Zubereitung nach Anspruch 1 oder 2, **dadurch gekennzeichnet, dass** sie 10 bis 80 Gew.-% Natrium-Chlorid-Eisen-Iod-Thermalwasser enthält.

4. Zubereitung nach einem der Ansprüche 1 bis 3, **dadurch gekennzeichnet, dass** die hydrophile Komponente, bezogen auf das Gewicht der Komponente, 25-50% Aqua (CAS-Nr. 7732-18-5), 10-25% Glycoproteins (CAS-Nr. 84604-16-0), 10-25% Panthenol (CAS-Nr. 81-13-0), 5-10% Propylene Glycol (CAS-Nr. 57-55-6), 1-5% Olea Europaea Leaf Extract (CAS-Nr. 84012-27-1), 1-5% Aspalathus Linearis Extract (CAS-Nr. 776295-36-4), 1-5% Centella Asiatica Extract (CAS-Nr. 84696-21-9), 1-5% Hamamelis Virginiana Extract (CAS-Nr. 84696-19-5), 1-5% Nelumbo Nucifera Extract (CAS-Nr. 85085-51-4), 1-5% Stevia Rebaudiana Extract (CAS-Nr. 91722-21-3) und < 0,1% Hyaluronic Acid (CAS-Nr. 9004-61-9) enthält.

5. Zubereitung nach Anspruch 4, **dadurch gekennzeichnet, dass** die hydrophile Komponente, bezogen auf das Gewicht der Komponente, 48,9400% Aqua (CAS-Nr. 7732-18-5), 20,0000% Glycoproteins (CAS-Nr. 84604-16-0), 10,0000% Panthenol (CAS-Nr. 81-13-0), 6,0000% Propylene Glycol (CAS-Nr. 57-55-6), 3,0000% Olea Europaea Leaf Extract (CAS-Nr. 84012-27-1), 3,0000% Aspalathus Linearis Extract (CAS-Nr. 776295-36-4), 3,0000% Centella Asiatica Extract (CAS-Nr. 84696-21-9), 2,2500% Hamamelis Virginiana Extract (CAS-Nr. 84696-19-5), 2,2500% Nelumbo Nucifera Extract (CAS-Nr. 85085-51-4), 1,5000% Stevia Rebaudiana Extract (CAS-Nr. 91722-21-3) und 0,0600% Hyaluronic Acid (CAS-Nr. 9004-61-9) enthält.

6. Zubereitung nach einem der Ansprüche 1 bis 5, **dadurch gekennzeichnet, dass** die hydrophile Komponente, bezogen auf das Gesamtgewicht der Zubereitung, in einer Menge von 2 bis 10% enthalten ist.

7. Zubereitung nach einem der Ansprüche 1 bis 6 , **dadurch gekennzeichnet, dass** die lipophile Komponente, bezogen auf das Gewicht der Komponente, 10-25% Macadamia Ternifolia Seed Oil (CAS-Nr. 128497-20-1/129811-19-4), 10-25% Simmondsia Chinensis Seed Oil (CAS-Nr. 61789-91-1), 10-25% Butyrospermum Parkii Butter (CAS-Nr. 91080-23-8), 10-25% Panthenyl Triacetate (CAS-Nr. 94089-18-6), 10-25% Farnesyl Acetate (CAS-Nr. 29548-30-9), 10-25% Farnesol (CAS-Nr. 4602-84-0), 5-10% Zea Mays Germ Oil (CAS-Nr. 8001-30-7), 5-10% Argania Spinosa Kernel Oil (CAS-Nr. 223747-87-3), 1-5% Triticum Vulgare Germ Oil (CAS-Nr. 68917-73-7), 1-5% Hordeum Vulgare Cera (CAS-Nr. 85251-64-5), < 0,1% Tocopherol (CAS-Nr. 10191-41-0) und < 0,1% Retinyl Palmitate (CAS-Nr. 79-81-2) enthält.

8. Zubereitung nach Anspruch 7, **dadurch gekennzeichnet, dass** die lipophile Komponente, bezogen auf das Gewicht der Komponente, 21,0000% Macadamia Ternifolia Seed Oil (CAS-Nr. 128497-20-1/129811-19-4), 15,0000% Simmondsia Chinensis Seed Oil (CAS-Nr. 61789-91-1), 13,0000% Butyrospermum Parkii Butter (CAS-Nr. 91080-23-8), 10,5400% Panthenyl Triacetate (CAS-Nr. 94089-18-6), 10,2300% Farnesyl Acetate (CAS-Nr. 29548-30-9), 10,2300% Farnesol (CAS-Nr. 4602-84-0), 9,0955% Zea Mays Germ Oil (CAS-Nr. 8001-30-7), 5,0000% Argania spinosa Kernel Oil (CAS-Nr. 223747-87-3), 3,8870% Triticum Vulgare Germ Oil (CAS-Nr. 68917-73-7), 2,0000% Hordeum Vulgare Cera (CAS-Nr. 85251-64-5), 0,0130% Tocopherol (CAS-Nr. 10191-41-0) und 0,0046% Retinyl Palmitate (CAS-Nr. 79-81-2) enthält.

9. Zubereitung nach einem der Ansprüche 1 bis 8, **dadurch gekennzeichnet, dass** sie mindestens einen Zusatzstoff ausgewählt aus der Gruppe bestehend aus Allantoin (CAS-Nr. 97-59-6), Adenosintriphosphat (CAS-Nr. 56-65-5), Betula Alba Leaf Extract (CAS-Nr. 84012-15-7), Biotin (CAS-Nr. 58-85-5), Citrus Grandis Oil (CAS-Nr. 8016-20-4), Cinchona Extract (CAS-Nr. 84776-28-3), Camelia Sinensis Extract (CAS-Nr. 84650-60-2), Chamomilla Recutita Extract (CAS-Nr. 84082-60-0), Equisetum Arvense Extract (CAS-Nr. 71011-23-9), Humulus Lupulus Extract (CAS-Nr. 8060-28-4), Niacinamid (CAS-Nr. 98-92-0), Niacin (CAS-Nr. 59-67-6), Panicum Miliaceum Extract (CAS-Nr. 90082-36-3), Salvia Officinalis Extract (CAS-Nr. 84082-79-1), Tilia Cordata Flower Extract (CAS-Nr. 84929-52-2), Urtica Dioica Extract (CAS-Nr. 84012-40-8) und Vanilla Planifolia Extract (CAS-Nr. 84650-63-5) enthält.

10. Zubereitung nach einem der Ansprüche 1 bis 9, **dadurch gekennzeichnet, dass** sie einen oder mehrere herkömmliche Hilfsstoffe enthält, die aus Emulgatoren, Co-Emulgatoren, Lösungsmitteln, Lösungsvermittlern, Konservierungsmitteln, Antioxidantien, Komplexbildnern, Feuchthaltemitteln, Filmbildnern, Gelbildnern, konsistenzregulierenden Mitteln, Puffern, Stabilisatoren, Antistatika, Lichtschutzfiltern, Färbemitteln, Desodorantien, Duftstoffen, Maskierungsmitteln, Tensiden, schaumregulierenden Mitteln und schaumstabilisierenden Mitteln ausgewählt sind.

11. Kosmetische Zubereitung nach einem der Ansprüche 1 bis 10, **dadurch gekennzeichnet, dass**
a) die kosmetische Zubrereitung in Form einer Creme vorliegt,
b) gegebenenfalls die als Creme vorliegende kosmetische Zubereitung, bezogen auf das Gesamtgewicht der Zubereitung, ≥50% Aqua (CAS-Nr. 7732-18-5), 5-10% Steareth-10 (CAS-Nr. 9005-00-9), 1-5% Myristyl Myristate (CAS-Nr. 3234-85-3), 1-5% Cetearyl Isononanoate (CAS-Nr. 84878-33-1), 1-5% Glyceryl Stearate (CAS-Nr. 31566-31-1), 1-5% Cetearyl Alcohol (CAS-Nr. 67762-27-0/8005-44-5), 1-5% Glycoproteins (CAS-Nr. 84604-16-0), 0,1-1% Macadamia Ternifolia Seed Oil (CAS-Nr. 128497-20-1/129811-19-4), 0,1-1% Simmondsia Chinensis Oil (CAS-Nr. 61789-91-1), 0,1-1% Panthenol (CAS-Nr. 81-13-0), 0,1-1% Butyrospermum Parkii Butter (CAS-Nr. 91080-23-8), 0,1-1% Panthenyl Triacetate (CAS-Nr. 94089-18-6), 0,1-1% Dimethicone (CAS-Nr. 9006-65-9/63148-62-9), 0,1-1% Farnesol, (CAS-Nr. 4602-84-0), 0,1-1% Farnesyl Acetate (CAS-Nr. 29548-30-9), 0,1-1% Zea mays Germ Oil (CAS-Nr. 8001-30-7), 0,1-1% Propylene Glycol (CAS-Nr. 57-55-6), 0,1-1% Phenoxyethanol (CAS-Nr. 122-99-6), 0,1-1% Imidazolidinyl Urea (CAS-Nr. 39236-46-9), 0,1-1% Argania Spinosa Kernel Oil (CAS-Nr. 223747-87-3), 0,1-1% Centella Asiatica Extract (CAS-Nr. 84696-21-9), 0,1-1% Olea Europaea Leaf Extract (CAS-Nr. 84012-27-1), 0,1-1% Aspalathus Linearis Extract (CAS-Nr. 776295-36-4), 0,1-1% Triticum Vulgare Germ Oil (CAS-Nr. 68917-73-7), 0,1-1% Parfum, 0,1-1% Nelumbo Nucifera Extract (CAS-Nr. 85085-51-4), 0,1-1% Hamamelis Virginiana Extract (CAS-Nr. 84696-19-5), 0,1-1% Sales, 0,1-1% Stevia Rebaudiana Extract (CAS-Nr. 91722-21-3), <0,1% Hordeum vulgare Cera (CAS-Nr. 85251-64-5), <0,1% Methylparaben (CAS-Nr. 99-76-3), <0,1% alpha-Isomethyl Ionone, <0,1% Linalool (CAS-Nr. 78-70-6), <0,1% Citric Acid (CAS-Nr. 77-92-9), <0,1% Ethylparaben (CAS-Nr. 120-47-8), <0,1% Propylparaben (CAS-Nr. 94-13-3), <0,1% Butylparaben (CAS-Nr. 94-26-8), <0,1% Hydroxycitronellal (CAS-Nr. 107-75-5), <0,1% Hydroxy Methylpentyl-3-cyclohexene-carboxaldehyde (CAS-Nr. 31906-04-4), <0,1% Hyaluronic Acid (CAS-Nr. 9004-61-9), <0,1% Citral (CAS-Nr. 5392-40-5) <0,1% Cinnamyl Alcohol (CAS-Nr. 104-54-1), <0,1% Amyl Cinnamal (CAS-Nr. 122-40-7), <0,1% Isobutylparaben (CAS-Nr. 857259), <0,1% Limonene (EINECS-Nr. 5989-27-5) und < 0,1% Tocopherol (CAS-Nr. 10191-41-0) enthält und
c) gegebenenfalls die als Creme vorliegende kosmetische Zubereitung, bezogen auf das Gesamtgewicht der Zubereitung, 6,019000% Steareth-10 (CAS-Nr. 9005-00-9), 4,000000% Myristyl Myristate (CAS-Nr. 3234-85-3), 2,990000% Cetearyl Isononanoate (CAS-Nr. 84878-33-1), 1,989000% Glyceryl Stearate (CAS-Nr. 31566-31-1), 1,521000% Cetearyl Alcohol (CAS-Nr. 67762-27-0/8005-44-5), 1,400000% Glycoproteins (CAS-Nr. 84604-16-0), 0,987000% Macadamia Ternifolia Seed Oil (CAS-Nr. 128497-20-1/129811-19-4), 0,705000% Simmondsia Chinensis Oil (CAS-Nr. 61789-91-1), 0,700000% Panthenol (CAS-Nr. 81-13-0), 0,611000% Butyrospermum Parkii Butter (CAS-Nr. 91080-23-8), 0,495380% Panthenyl Triacetate (CAS-Nr. 94089-18-6), 0,481000% Dimethicone (CAS-Nr. 9006-65-9/63148-62-9), 0,480823% Farnesol (CAS-Nr. 4602-84-0), 0,480810% Farnesyl Acetate (CAS-Nr. 29548-30-9), 0,427486% Zea Mays Germ Oil (CAS-Nr. 8001-30-7), 0,420000% Propylene Glycol (CAS-Nr. 57-55-6), 0,370000% Phenoxyethanol (CAS-Nr. 122-99-6), 0,300000% Imidazolidinyl Urea (CAS-Nr. 39236-46-9), 0,235000% Argania Spinosa Kernel Oil (CAS-Nr. 223747-87-3), 0,210000% Centella Asiatica Extract (CAS-Nr. 84696-21-9), 0,210000% Olea Europaea Leaf Extract (CAS-Nr. 84012-27-1), 0,210000% Aspalathus Linearis Extract (CAS-Nr. 776295-36-4), 0,182689% Triticum Vulgare Germ Oil (CAS-Nr. 68917-73-7), 0,176213% Parfum, 0,157500% Nelumbo Nucifera Extract (CAS-Nr. 85085-51-4), 0,157500% Hamamelis Virginiana Extract (CAS-Nr. 84696-19-5), 0,150000% Sales, 0,105000% Stevia Rebaudiana Extract (CAS-Nr. 91722-21-3), 0,094000% Hordeum Vulgare Cera (CAS-Nr. 85251-64-5), 0,085000% Methylparaben (CAS-Nr. 99-76-3), 0,025575% alpha-Isomethyl lonone, 0,023500% Linalool (CAS-Nr. 78-70-6), 0,020000% Citric Acid (CAS-Nr. 77-92-9), 0,020000% Ethylparaben (CAS-Nr. 120-47-8), 0,015000% Propylparaben (CAS-Nr. 94-13-3), 0,007500% Butylparaben (CAS-Nr. 94-26-8), 0,006825% Hydroxycitronellal (CAS-Nr. 107-75-5), 0,004500% Hydroxy Methylpentyl-3-cyclohexene-carboxaldehyde (CAS-Nr. 31906-04-4), 0,004200% Hyaluronic Acid (CAS-Nr. 9004-61-9), 0,004075% Citral (CAS-Nr. 5392-40-5), 0,003000% Cinnamyl Alcohol (CAS-Nr. 104-54-1), 0,002875% Amyl Cinnamal (CAS-Nr. 122-40-7), 0,002500% Isobutylparaben (CAS-Nr. 857259), 0,001750% Limonene (EINECS-Nr. 5989-27-5), 0,000611% Tocopherol (CAS-Nr. 10191-41-0) und Wasser enthält.

12. Zubereitung nach einem der Ansprüche 1 bis 10, **dadurch gekennzeichnet, dass**
a) die kosmetische Zubrereitung in Form einer Körperlotion vorliegt,
b) gegebenenfalls die als Lotion vorliegende kosmetische Zubereitung, bezogen auf das Gesamtgewicht der Zubereitung, ≥75% Aqua (CAS-Nr. 7732-18-5), 1-5% Glycerin (CAS-Nr. 56-81-5), 1-5% Zea Mays Germ Oil (CAS-Nr. 8001-30-7), 1-5% Caprylic/Capric Triglyceride (CAS-Nr. 73398-61-5/65381-09-1), 0,1-1% Polysorbate 85 (CAS-Nr. 9005-70-3), 0,1-1% Macadamia Ternifolia Seed Oil (CAS-Nr. 128497-20-1/129811-19-4), 0,1-1% Propylene Glycol (CAS-Nr. 57-55-6), 0,1-1% Glycoproteins (CAS-Nr. 84604-16-0), 0,1-1% Simmondsia Chinensis Oil (CAS-Nr. 61789-91-1), 0,1-1% Triticum Vulgare Germ Oil (CAS-Nr. 68917-73-7), 0,1-1% Saccharide Isomerate (CAS-Nr. 100843-69-4), 0,1-1% Acrylates/Acrylamide Copolymer, 0,1-1% Petrolatum (8009-03-08), 0,1-1% Panthenol (CAS-Nr. 81-13-0), 0,1-1% Calendula Officinalis Extract (CAS-Nr. 84776-23-8), 0,1-1% Chamomilla Recutita Extract (CAS-Nr.84082-60-0), 0,1-1% Butyrospermum Parkii Butter (CAS-Nr. 91080-23-8), 0,1-1% Panthenyl Triacetate (CAS-Nr. 94089-18-6), 0,1-1% Farnesyl Acetate (CAS-Nr. 29548-30-9), 0,1-1% Farnesol (CAS-Nr. 4602-84-0), 0,1-1% Imidazolidinyl Urea (CAS-Nr. 39236-46-9), 0,1-1% Allantoin (CAS-Nr. 97-59-6), 0,1-1% Carbomer (CAS-Nr. 9007-20-9/9003-01-4), 0,1-1% Sales, 0,1-1% Argania spinosa Kernel Oil (CAS-Nr. 223747-87-3), 0,1-1% Olea Europaea Leaf Extract (CAS-Nr. 84012-27-1), 0,1-1% Aspalathus Linearis Extract (CAS-Nr. 776295-36-4), 0,1-1% Centella Asiatica Extract (CAS-Nr. 84696-21-9), 0,1-1% Parfum, 0,1-1% Methylparaben (CAS-Nr. 99-76-3), < 0,1% Nelumbo Nucifera Extract (CAS-Nr. 85085-51-4), < 0,1% Hamamelis Virginiana Extract (CAS-Nr. 84696-19-5), <0,1% Hordeum Vulgare Cera (CAS-Nr. 85251-64-5), < 0,1% Stevia Rebaudiana Extract (CAS-Nr. 91722-21-3), <0,1% Propylparaben (CAS-Nr. 94-13-3), <0,1% Citrus Grandis Oil (CAS-Nr. 8016-20-4), < 0,1% Vanilla Planifolia Extract (CAS-Nr. 84650-63-5), < 0,1% Elettaria Cardamon Oil (CAS-Nr. 8000-66-6), <0,1% Hexyl Cinnamal (CAS-Nr. 101-86-0), <0,1% Benzyl Benzoate (CAS-Nr. 120-51-4), <0,1% Linalool (CAS-Nr. 78-70-6), <0,1% Limonene, <0,1% Hyaluronic Acid (CAS-Nr. 9004-61-9), <0,1% alpha-Isomethyl Ionone, <0,1% Tocopherol (CAS-Nr. 10191-41-0), <0,1% Citronellol (CAS-Nr. 106-22-9) und < 0,1% Retinyl Palmitate (CAS-Nr. 79-81-2) enthält und
c) gegebenenfalls die als Lotion vorliegende kosmetische Zubereitung, bezogen auf das Gesamtgewicht der Zubereitung, 1,70000% Glycerin (CAS-Nr. 56-81-5), 1,35232% Zea Mays Germ Oil (CAS-Nr. 8001-30-7), 1,24196% Caprylic/Capric Triglyceride (CAS-Nr. 73398-61-5/65381-09-1), 0,96000% Polysorbate 85 (CAS-Nr. 9005-70-3), 0,83000% Macadamia Ternifolia Seed Oil (CAS-Nr. 128497-20-1/129811-19-4), 0,80000% Propylene Glycol (CAS-Nr. 57-55-6), 0,80000% Glycoproteins (CAS-Nr. 84604-16-0), 0,65000% Simmondsia Chinensis Oil (CAS-Nr. 61789-91-1), 0,59277% Triticum Vulgare Germ Oil (CAS-Nr. 68917-73-7), 0,55000% Saccharide Isomerate (CAS-Nr. 100843-69-4), 0,54000% Acrylates/Acrylamide Copolymer, 0,50000% Petrolatum (8009-03-08), 0,40000% Panthenol (CAS-Nr. 81-13-0), 0,40000% Calendula Officinalis Extract (CAS-Nr. 84776-23-8), 0,40000% Chamomilla Recutita Extract (CAS-Nr.84082-60-0), 0,39000% Butyrospermum Parkii Butter (CAS-Nr. 91080-23-8), 0,31620% Panthenyl Triacetate (CAS-Nr. 94089-18-6), 0,30690% Farnesyl Acetate (CAS-Nr. 29548-30-9), 0,30690% Farnesol (CAS-Nr. 4602-84-0), 0,30000% Imidazolidinyl Urea (CAS-Nr. 39236-46-9), 0,30000% Allantoin (CAS-Nr. 97-59-6), 0,25000% Carbomer (CAS-Nr. 9007-20-9/9003-01-4), 0,15000% Sales, 0,15000% Argania Spinosa Kernel Oil (CAS-Nr. 223747-87-3), 0,12000% Olea Europaea Leaf Extract (CAS-Nr. 84012-27-1), 0,12000% Aspalathus Linearis Extract (CAS-Nr. 776295-36-4), 0,12000% Centella Asiatica Extract (CAS-Nr. 84696-21-9), 0,11642% Parfum, 0,11000% Methylparaben (CAS-Nr. 99-76-3), 0,09000% Nelumbo Nucifera Extract (CAS-Nr. 85085-51-4), 0,09000% Hamamelis Virginiana Extract (CAS-Nr. 84696-19-5), 0,06000% Hordeum Vulgare Cera (CAS-Nr. 85251-64-5), 0,06000% Stevia Rebaudiana Extract (CAS-Nr. 91722-21-3), 0,03000% Propylparaben (CAS-Nr. 94-13-3), 0,02000% Citrus Grandis Oil (CAS-Nr. 8016-20-4), 0,01600% Vanilla Planifolia Extract (CAS-Nr. 84650-63-5), 0,01500% Elettaria Cardamon Oil (CAS-Nr. 8000-66-6), 0,00860% Hexyl Cinnamal (CAS-Nr. 101-86-0), 0,00730% Benzyl Benzoate (CAS-Nr. 120-51-4), 0,00600% Linalool (CAS-Nr. 78-70-6), 0,00417% Limonene, 0,00240% Hyaluronic Acid (CAS-Nr. 9004-61-9), 0,00200% alpha-Isomethyl lonone, 0,00239% Tocopherol (CAS-Nr. 10191-41-0), 0,00180% Citronelfol (CAS-Nr. 106-22-9), 0,00056% Retinyl Palmitate (CAS-Nr. 79-81-2) und Wasser enthält.

13. Zubereitung nach einem der Ansprüche 1 bis 10, **dadurch gekennzeichnet, dass**
a) die kosmetische Zubrereitung in Form eines Haartonikums vorliegt,
b) gegebenenfalls die als Haartonikum vorliegende kosmetische Zubereitung, bezogen auf das Gesamtgewicht der Zubereitung, ≥ 50% Aqua (CAS-Nr. 7732-18-5), 10-25% Mipa-Laureth Sulfate (CAS-Nr. 83016-76-6/9062-04-8), 1-5% Mipa-Lauryl Sulfate (CAS-Nr. 21142-28-9), 1-5% Cocoamidopropyl Betain (CAS-Nr. 263-058-8), 1-5% Disodium Laureth Sulfosuccinate (CAS-Nr. 39354-45-5), 1-5% PEG-12 Glyceryl Laurate (CAS-Nr. 59070-56-3), 1-5% Disodium Cocoamphodiacetate (CAS-Nr. 68650-39-5), 1-5% Laureth-13 (CAS-Nr. 9002-92-0), 1-5% Glycoproteins (CAS-Nr. 84604-16-0), 0,1-1% PEG-36 Castor Oil (CAS-Nr. 61791-12-6), 0,1-1% Propylene Glycol (CAS-Nr. 57-55-6), 0,1-1% Butylene Glycol (CAS-Nr. 107-88-0), 0,1-1% Panthenol (CAS-Nr. 81-13-0), 0,1-1% Linoleamide DEA (CAS-Nr. 56863-02-6), 0,1-1 % Farnesyl Acetate (CAS-Nr. 29548-30-9), 0,1-1% Farnesol (CAS-Nr. 4602-84-0), 0,1-1% Parfum, 0,1-1% PEG-10 Olive Glycerides, 0,1-1% Imidazolidinyl Urea (CAS-Nr. 39236-46-9), 0,1-1% Hydrolyzed Milk Protein (CAS-Nr. 92797-39-2/8049-98-7), 0,1-1% Olea Europaea Leaf Extract (CAS-Nr. 84012-27-1), 0,1-1% Centella Asiatica Extract (CAS-Nr. 84696-21-9), 0,1-1% Aspalathus Linearis Extract (CAS-Nr. 776295-36-4), 0,1-1% Sales, 0,1-1% Nelumbo Nucifera Extract (CAS-Nr. 85085-51-4), 0,1-1% Hamamelis Virginiana Extract (CAS-Nr. 84696-19-5), 0,1-1% Methyl-paraben (CAS-Nr. 99-76-3), 0,1-1% Panicum Miliaceum Extract (CAS-Nr. 90082-36-3), 0,1-1% Salvia Officinalis Extract (CAS-Nr. 84082-79-1), 0,1-1% Adenosine Triphosphate (CAS-Nr. 56-65-5), 0,1-1% Betula Alba Leaf Extract (CAS-Nr. 84012-15-7), 0,1-1% Polyquaternium-10 (CAS-Nr. 81859-24-7/53568-66-4), 0,1-1% Chamomilla Recutita Extract (CAS-Nr. 84082-60-0), 0,1-1% Niacinamide (CAS-Nr. 98-92-0), <0,1% Camelia Sinensis Extract (CAS-Nr. 84650-60-2), <0,1% Tilia Cordata Flower Extract (CAS-Nr. 84929-52-2), <0,1% Urtica Dioica Extract (CAS-Nr. 84012-40-8), <0,1% Panthenyl Triacetate (CAS-Nr. 94089-18-6), <0,1% Humulus Lupulus Extract (CAS-Nr. 8060-28-4), <0,1% Cinchona Pubescens Extract (CAS-Nr. 84776-28-3), < 0,1% Equisetum Arvense Extract (CAS-Nr. 71011-23-9), <0,1% Stevia Rebaudiana Extract (CAS-Nr. 91722-21-3), <0,1% Citrus Grandis Oil (CAS-Nr. 8016-20-4), < 0,1% Vanilla Planifolia Extract (CAS-Nr. 84650-63-5), <0,1% Elettaria Cardamon Oil (CAS-Nr. 8000-66-6), <0,1% Propylparaben (CAS-Nr. 94-13-3), <0,1% Hexyl Cinnamal (CAS-Nr. 101-86-0), <0,1% Benzyl Benzoate (CAS-Nr. 120-51-4), <0,1% Linalool (CAS-Nr. 78-70-6), <0,1% Limonene, <0,1% Niacin (CAS-Nr. 59-67-6), <0,1% Hyaluronic Acid (CAS-Nr. 9004-61-9) und < 0,1% Biotin (CAS-Nr. 58-85-5) enthält und
c) gegebenenfalls die als Haartonikum vorliegende kosmetische Zubereitung, bezogen auf das Gesamtgewicht der Zubereitung, 10,00000% Mipa-Laureth Sulfate (CAS-Nr. 83016-76-6/9062-04-8), 3,15000% Mipa-Lauryl Sulfate (CAS-Nr. 21142-28-9), 1,60000% Cocoamidopropyl Betain (CAS-Nr. 263-058-8), 1,50000% Disodium Laureth Sulfosuccinate (CAS-Nr. 39354-45-5), 1,20000% PEG-12 Glyceryl Laurate (CAS-Nr. 59070-56-3), 1,20000% Disodium Cocoamphodiacetate (CAS-Nr. 68650-39-5), 1,20000% Laureth-13 (CAS-Nr. 9002-92-0), 1,00000% Glycoproteins (CAS-Nr. 84604-16-0), 0,90000% PEG-36 Castor Oil (CAS-Nr. 61791-12-6), 0,86000% Propylene Glycol (CAS-Nr. 57-55-6), 0,60000% Butylene Glycol (CAS-Nr. 107-88-0), 0,54990% Panthenol (CAS-Nr. 81-13-0), 0,45000% Linoleamide DEA (CAS-Nr. 56863-02-6), 0,45000% Farnesyl Acetate (CAS-Nr. 29548-30-9), 0,36000% Farnesol (CAS-Nr. 4602-84-0), 0,34927% Parfum, 0,30000% PEG-10 Olive Glycerides, 0,30000% Imidazolidinyl Urea (CAS-Nr. 39236-46-9), 0,20000% Hydrolyzed Milk Protein (CAS-Nr. 92797-39-2/8049-98-7), 0,15000% Olea Europaea Leaf Extract (CAS-Nr. 84012-27-1), 0,15000% Centella Asiatica Extract (CAS-Nr. 84696-21-9), 0,15000% Aspalathus Linearis Extract (CAS-Nr. 776295-36-4), 0,15000% Sales, 0,11250% Nelumbo Nucifera Extract (CAS-Nr. 85085-51-4), 0,11250% Hamamelis Virginiana Extract (CAS-Nr. 84696-19-5), 0,11000% Methylparaben (CAS-Nr. 99-76-3), 0,10000% Panicum Miliaceum Extract (CAS-Nr. 90082-36-3), 0,10000% Salvia Officinalis Extract (CAS-Nr. 84082-79-1), 0,10000% Adenosine Triphosphate (CAS-Nr. 56-65-5), 0,10000% Betula Alba Leaf Extract (CAS-Nr. 84012-15-7), 0,10000% Polyquaternium-10 (CAS-Nr. 81859-24-7/53568-66-4), 0,10000% Chamomilla Recutita Extract (CAS-Nr. 84082-60-0), 0,10000% Niacinamide (CAS-Nr. 98-92-0), 0,09000% Camelia Sinensis Extract (CAS-Nr. 84650-60-2), 0,09000% Tilia Cordata Flower Extract (CAS-Nr. 84929-52-2), 0,09000% Urtica Dioica Extract (CAS-Nr. 84012-40-8), 0,09000% Panthenyl Triacetate (CAS-Nr. 94089-18-6), 0,09000% Humulus Lupulus Extract (CAS-Nr. 8060-28-4), 0,09000% Cinchona Pubescens Extract (CAS-Nr. 84776-28-3), 0,09000% Equisetum Arvense Extract (CAS-Nr. 71011-23-9), 0,07500% Stevia Rebaudiana Extract (CAS-Nr. 91722-21-3), 0,06000% Citrus Grandis Oil (CAS-Nr. 8016-20-4), 0,04800% Vanilla Planifolia Extract (CAS-Nr. 84650-63-5), 0,04500% Elettaria Cardamon Oil (CAS-Nr. 8000-66-6), 0,03000% Propylparaben (CAS-Nr. 94-13-3), 0,02580% Hexyl Cinnamal (CAS-Nr. 101-86-0), 0,02190% Benzyl Benzoate (CAS-Nr. 120-51-4), 0,01801% Linalool (CAS-Nr. 78-70-6), 0,01252% Limonene, 0,01000% Niacin (CAS-Nr. 59-67-6), 0,00300% Hyaluronic Acid (CAS-Nr. 9004-61-9), 0,00010% Biotin (CAS-Nr. 58-85-5) und Wasser enthält.

14. Zubereitung nach einem der Ansprüche 1 bis 10, **dadurch gekennzeichnet, dass**
a) die kosmetische Zubrereitung in Form eines Dusch-Shampoos vorliegt,
b) gegebenenfalls die als Dusch-Shampoo vorliegende kosmetische Zubereitung, bezogen auf das Gesamtgewicht der Zubereitung, ≥ 50% Aqua (CAS-Nr. 7732-18-5), 10-25% Mipa-Laureth Sulfate (CAS-Nr. 83016-76-6/9062-04-8), 1-5% Mipa-Lauryl Sulfate (CAS-Nr. 21142-28-9), 1-51% Cocoamidopropyl Betain (CAS-Nr. 263-058-8), 1-5% Disodium Laureth Sulfosuccinate (CAS-Nr. 39354-45-5), 1-5% Laureth-13 (CAS-Nr. 9002-92-0), 1-5% Disodium Cocoamphodiacetate (CAS-Nr. 68650-39-5), 0,1-1% Sodium Chloride (CAS-Nr. 7647-14-5), 0,1-1% Propylene Glycol (CAS-Nr. 57-55-6), 0,1-1% Glycoproteins (CAS-Nr. 84604-16-0), 0,1-1% Linoleamide DEA (CAS-Nr. 56863-02-6), 0,1-1% Panthenol (CAS-Nr. 81-13-0), 0,1-1% Polyquaternium-10 (CAS-Nr. 81859-24-7/53568-66-4), 0,1-1% PEG-10 Olive Glycerides, 0,1-1% Imidazolidinyl Urea (CAS-Nr. 39236-46-9), 0,1-1% Panicum Miliaceum Extract (CAS-Nr. 90082-36-3), 0,1-1% Chamomilla Recutita Extract (CAS-Nr. 84082-60-0), 0,1-1% Salvia Officinalis Extract (CAS-Nr. 84082-79-1), 0,1-1% Betula Alba Leaf Extract (CAS-Nr. 84012-15-7), 0,1-1% Parfum, 0,1-1% Tilia cordata Flower Extract (CAS-Nr. 84929-52-2), 0,1-1% Urtica Dioica Extract (CAS-Nr. 84012-40-8), 0,1-1% Camelia Sinensis Extract (CAS-Nr. 84650-60-2), 0,1-1% Equisetum Arvense Extract (CAS-Nr. 71011-23-9), 0,1-1% Humulus Lupulus Extract (CAS-Nr. 8060-28-4), 0,1-1% Cinchona Extract (CAS-Nr. 84776-28-3), 0,1-1% Sales, 0,1-1% PEG-12 Glyceryl Laurate (CAS-Nr. 59070-56-3), 0,1-1% Methylparaben (CAS-Nr. 99-76-3), <0,1% PEG-36 Castor Oil (CAS-Nr. 61791-12-6), <0,1% Centella Asiatica Extract (CAS-Nr. 84696-21-9), <0,1% Olea Europaea Leaf Extract (CAS-Nr. 84012-27-1), <0,1% Aspalathus Linearis Extract (CAS-Nr. 776295-36-4), < 0,1% Hamamelis Virginiana Extract (CAS-Nr. 84696-19-5), < 0,1% Nelumbo Nucifera Extract (CAS-Nr. 85085-51-4), < 0,1% Farnesyl Acetate (CAS-Nr. 29548-30-9), <0,1% Citrus Grandis Oil (CAS-Nr. 8016-20-4), <0,1% Stevia Rebaudiana Extract (CAS-Nr. 91722-21-3), <0,1% Farnesol (CAS-Nr. 4602-84-0), < 0,1% Vanilla Planifolia Extract (CAS-Nr. 84650-63-5), < 0,1% Elettaria Cardamon Oil (CAS-Nr. 8000-66-6), <0,1% Propylparaben (CAS-Nr. 94-13-3), <0,1% Niacin (CAS-Nr. 59-67-6), <0,1% Hexyl Cinnamal (CAS-Nr. 101-86-0), <0,1% Benzyl Benzoate (CAS-Nr. 120-51-4), <0,1% Linalool (CAS-Nr. 78-70-6) und <0,1% Hyaluronic Acid (CAS-Nr. 9004-61-9) enthält und
c) gegebenenfalls die als Dusch-Shampoo vorliegende kosmetische Zubereitung, bezogen auf das Gesamtgewicht der Zubereitung, 10,00000% Mipa-Laureth Sulfate (CAS-Nr. 83016-76-6/9062-04-8), 3,15000% Mipa-Lauryl Sulfate (CAS-Nr. 21142-28-9), 1,60000% Cocoamidopropyl Betain (CAS-Nr. 263-058-8), 1,50000% Disodium Laureth Sulfosuccinate (CAS-Nr. 39354-45-5), 1,20000% Laureth-13 (CAS-Nr. 9002-92-0), 1,20000% Disodium Cocoamphodiacetate (CAS-Nr. 68650-39-5), 0,90000% Sodium Chloride (CAS-Nr. 7647-14-5), 0,71000% Propylene Glycol (CAS-Nr. 57-55-6), 0,50000% Glycoproteins (CAS-Nr. 84604-16-0), 0,45000% Linoleamide DEA (CAS-Nr. 56863-02-6), 0,37475% Panthenol (CAS-Nr. 81-13-0), 0,30000% Polyquaternium-10 (CAS-Nr. 81859-24-7/53568-66-4), 0,30000% PEG-10 Olive Glycerides, 0,30000% Imidazolidinyl Urea (CAS-Nr. 39236-46-9), 0,25000% Panicum Miliaceum Extract (CAS-Nr. 90082-36-3), 0,25000% Chamomilla Recutita Extract (CAS-Nr. 84082-60-0), 0,25000% Salvia Officinalis Extract (CAS-Nr. 84082-79-1), 0,25000% Betula Alba Leaf Extract (CAS-Nr. 84012-15-7), 0,23285% Parfum, 0,22500% Tilia Cordata Flower Extract (CAS-Nr. 84929-52-2), 0,22500% Urtica Dioica Extract (CAS-Nr. 84012-40-8), 0,22500% Camelia Sinensis Extract (CAS-Nr. 84650-60-2), 0,22500% Equisetum Arvense Extract (CAS-Nr. 71011-23-9), 0,22500% Humulus Lupulus Extract (CAS-Nr. 8060-28-4), 0,22500% Cinchona Extract (CAS-Nr. 84776-28-3), 0,15000% Sales, 0,12000% PEG-12 Glyceryl Laurate (CAS-Nr. 59070-56-3), 0,11000% Methylparaben (CAS-Nr. 99-76-3), 0,09000% PEG-36 Castor Oil (CAS-Nr. 61791-12-6), 0,075001% Centella Asiatica Extract (CAS-Nr. 84696-21-9), 0,07500% Olea Europaea Leaf Extract (CAS-Nr. 84012-27-1), 0,07500% Aspalathus Linearis Extract (CAS-Nr. 776295-36-4), 0,05625% Hamamelis Virginiana Extract (CAS-Nr. 84696-19-5), 0,05625% Nelumbo Nucifera Extract (CAS-Nr. 85085-51-4), 0,04500% Farnesyl Acetate (CAS-Nr. 29548-30-9), 0,04000% Citrus Grandis Oil (CAS-Nr. 8016-20-4), 0,03750% Stevia Rebaudiana Extract (CAS-Nr. 91722-21-3), 0,03600% Farnesol (CAS-Nr. 4602-84-0), 0,03200% Vanilla Planifolia Extract (CAS-Nr. 84650-63-5), 0,03000% Elettaria Cardamon Oil (CAS-Nr. 8000-66-6), 0,03000% Propylparaben (CAS-Nr. 94-13-3), 0,02500% Niacin (CAS-Nr. 59-67-6), 0,01720% Hexyl Cinnamal (CAS-Nr. 101-86-0), 0,01460% Benzyl Benzoate (CAS-Nr. 120-51-4), 0,01201% Linalool (CAS-Nr. 78-70-6), 0,00150% Hyaluronic Acid (CAS-Nr. 9004-61-9) und Wasser enthält.

15. Verwendung eines Natrium-Chlorid-Eisen-Iod-Thermalwassers mit einer Elektrolytsumme von mindestens 25 g/kg, bevorzugt mindestens 30 g/kg, und einem Eisengehalt von mindestens 9 mg/kg zur Herstellung einer kosmetischen Zubereitung zur Hautpflege gemäß einem der Ansprüche 1 bis 14.

## Claims

1. A cosmetic preparation for skin care, **characterized in that** in addition to conventional cosmetic excipients and additives, it contains, with respect to the total weight of the preparation,
(a) at least 2% of a hydrophilic component which contains Aqua (CAS No. 7732-18-5), Glycoproteins (CAS No. 84604-16-0), Panthenol (CAS No. 81-13-0), Propylene Glycol (CAS No. 57-55-6), Olea Europaea Leaf Extract (CAS No. 84012-27-1), Aspalathus Linearis Extract (CAS No. 776295-36-4), Centella Asiatica Extract (CAS No. 84696-21-9), Hamamelis Virginiana Extract (CAS No. 84696-19-5), Nelumbo Nucifera Extract (CAS No. 85085-51-4), Stevia Rebaudiana Extract (CAS No. 91722-21-3) and Hyaluronic Acid (CAS No. 9004-61-9),
(b) at least 10% of sodium-chloride-iron-iodine thermal water with an electrolyte concentration of at least 25 g/kg, preferably at least 30 g/kg, and an iron content of at least 9 mg/kg,
as well as, where appropriate,
(c) up to 15% of a lipophilic component which contains one or more components selected from the group consisting of Macadamia Ternifolia Seed Oil (CAS No. 128497-20-1/129811-19-4), Simmondsia Chinensis Seed Oil (CAS No. 61789-91-1), Butyrospermum Parkii Butter (CAS No. 91080-23-8), Panthenyl Triacetate (CAS No. 94089-18-6), Farnesyl Acetate (CAS No. 29548-30-9), Farnesol (CAS No. 4602-84-0), Zea Mays Germ Oil (CAS No. 8001-30-7), Argania Spinosa Kernel Oil (CAS No. 223747-87-3), Triticum Vulgare Germ Oil (CAS No. 68917-73-7), Hordeum Vulgare Cera (CAS No. 85251-64-5), Tocopherol (CAS No. 10191-41-0) and Retinyl Palmitate (CAS No. 79-81-2).

2. The preparation as claimed in claim 1, **characterized in that** the sodium-chloride-iron-iodine thermal water contains an electrolyte concentration of at least 37 g/kg.

3. The preparation as claimed in claim 1 or claim 2, **characterized in that** it contains 10% to 80% by weight of sodium-chloride-iron-iodine thermal water.

4. The preparation as claimed in one of claims 1 to 3, **characterized in that** the hydrophilic component contains, with respect to the weight of the component, 25-50% Aqua (CAS No. 7732-18-5), 10-25% Glycoproteins (CAS No. 84604-16-0), 10-25% Panthenol (CAS No. 81-13-0), 5-10% Propylene Glycol (CAS No. 57-55-6), 1-5% Olea Europaea Leaf Extract (CAS No. 84012-27-1), 1-5% Aspalathus Linearis Extract (CAS No. 776295-36-4), 1-5% Centella Asiatica Extract (CAS No. 84696-21-9), 1-5% Hamamelis Virginiana Extract (CAS No. 84696-19-5), 1-5% Nelumbo Nucifera Extract (CAS No. 85085-51-4), 1-5% Stevia Rebaudiana Extract (CAS No. 91722-21-3) and < 0.1% Hyaluronic Acid (CAS No. 9004-61-9).

5. The preparation as claimed in claim 4, **characterized in that** the hydrophilic component contains, with respect to the weight of the component, 48.9400% Aqua (CAS No. 7732-18-5), 20.0000% Glycoproteins (CAS No. 84604-16-0), 10.0000% Panthenol (CAS No. 81-13-0), 6.0000% Propylene Glycol (CAS No. 57-55-6), 3.0000% Olea Europaea Leaf Extract (CAS No. 84012-27-1), 3.0000% Aspalathus Linearis Extract (CAS No. 776295-36-4), 3.0000% Centella Asiatica Extract (CAS No. 84696-21-9), 2.2500% Hamamelis Virginiana Extract (CAS No. 84696-19-5), 2.2500% Nelumbo Nucifera Extract (CAS No. 85085-51-4), 1.5000% Stevia Rebaudiana Extract (CAS No. 91722-21-3) and 0.0600% Hyaluronic Acid (CAS No. 9004-61-9).

6. The preparation as claimed in one of claims 1 to 5, **characterized in that** it contains the hydrophilic component in an amount of 2% to 10% with respect to the total weight of the preparation.

7. The preparation as claimed in one of claims 1 to 6, **characterized in that** the lipophilic component contains, with respect to the weight of the component, 10-25% Macadamia Ternifolia Seed Oil (CAS No. 128497-20-1/129811-19-4), 10-25% Simmondsia Chinensis Seed Oil (CAS No. 61789-91-1), 10-25% Butyrospermum Parkii Butter (CAS No. 91080-23-8), 10-25% Panthenyl Triacetate (CAS No. 94089-18-6), 10-25% Farnesyl Acetate (CAS No. 29548-30-9), 10-25% Farnesol (CAS No. 4602-84-0), 5-10% Zea Mays Germ Oil (CAS No. 8001-30-7), 5-10% Argania Spinosa Kernel Oil (CAS No. 223747-87-3), 1-5% Triticum Vulgare Germ Oil (CAS No. 68917-73-7), 1-5% Hordeum Vulgare Cera (CAS No. 85251-64-5), < 0.1% Tocopherol (CAS No. 10191-41-0) and < 0.1% Retinyl Palmitate (CAS No. 79-81-2).

8. The preparation as claimed in claim 7, **characterized in that** the lipophilic component contains, with respect to the weight of the component, 21.0000% Macadamia Ternifolia Seed Oil (CAS No. 128497-20-1/129811-19-4), 15.0000% Simmondsia Chinensis Seed Oil (CAS No. 61789-91-1), 13.0000% Butyrospermum Parkii Butter (CAS No. 91080-23-8), 10.5400% Panthenyl Triacetate (CAS No. 94089-18-6), 10.2300% Farnesyl Acetate (CAS No. 29548-30-9), 10.2300% Farnesol (CAS No. 4602-84-0), 9.0955% Zea Mays Germ Oil (CAS No. 8001-30-7), 5.0000% Argania Spinosa Kernel Oil (CAS No. 223747-87-3), 3.8870% Triticum Vulgare Germ Oil (CAS No. 68917-73-7), 2.0000% Hordeum Vulgare Cera (CAS No. 85251-64-5), 0.0130% Tocopherol (CAS No. 10191-41-0) and 0.0046% Retinyl Palmitate (CAS No. 79-81-2).

9. The preparation as claimed in one of claims 1 to 8, **characterized in that** it contains at least one additive selected from the group consisting of Allantoin (CAS No. 97-59-6), Adenosine Triphosphate (CAS No. 56-65-5), Betula Alba Leaf Extract (CAS No. 84012-15-7), Biotin (CAS No. 58-85-5), Citrus Grandis Oil (CAS No. 8016-20-4), Cinchona Extract (CAS No. 84776-28-3), Camelia Sinensis Extract (CAS No. 84650-60-2), Chamomilla Recutita Extract (CAS No. 84082-60-0), Equisetum Arvense Extract (CAS No. 71011-23-9), Humulus Lupulus Extract (CAS No. 8060-28-4), Niacinamide (CAS No. 98-92-0), Niacin (CAS No. 59-67-6), Panicum Miliaceum Extract (CAS No. 90082-36-3), Salvia Officinalis Extract (CAS No. 84082-79-1), Tilia Cordata Flower Extract (CAS No. 84929-52-2), Urtica Dioica Extract (CAS No. 84012-40-8) and Vanilla Planifolia Extract (CAS No. 84650-63-5).

10. The preparation as claimed in one of claims 1 to 9, **characterized in that** it contains one or more conventional excipients selected from emulsifiers, co-emulsifiers, solvents, solubilizing agents, preservatives, antioxidants, complexing agents, humectants, film-forming agents, gel-forming agents, consistency regulating agents, buffers, stabilizers, antistatic agents, sunscreens, colorants, deodorants, fragrances, sequestering agents, surfactants, foam-regulating agents and foam-stabilizing agents.

11. The cosmetic preparation as claimed in one of claims 1 to 10, **characterized in that**
a) the cosmetic preparation is in the form of a cream,
b) where appropriate, the cosmetic preparation in the form of a cream contains, with respect to the total weight of the preparation, ≥ 50% Aqua (CAS No. 7732-18-5), 5-10% Steareth-10 (CAS No. 9005-00-9), 1-5% Myristyl Myristate (CAS No. 3234-85-3), 1-5% Cetearyl Isononanoate (CAS No. 84878-33-1), 1-5% Glyceryl Stearate (CAS No. 31566-31-1), 1-5% Cetearyl Alcohol (CAS No. 67762-27-0/8005-44-5), 1-5% Glycoproteins (CAS No. 84604-16-0), 0.1-1% Macadamia Ternifolia Seed Oil (CAS No. 128497-20-1/129811-19-4), 0.1-1% Simmondsia Chinensis Oil (CAS No. 61789-91-1), 0.1-1% Panthenol (CAS No. 81-13-0), 0.1-1% Butyrospermum Parkii Butter (CAS No. 91080-23-8), 0.1-1% Panthenyl Triacetate (CAS No. 94089-18-6), 0.1-1% Dimethicone (CAS No. 9006-65-9/63148-62-9), 0.1-1% Farnesol (CAS No. 4602-84-0), 0.1-1% Farnesyl Acetate (CAS No. 29548-30-9), 0.1-1% Zea Mays Germ Oil (CAS No. 8001-30-7), 0.1-1% Propylene Glycol (CAS No. 57-55-6), 0.1-1% Phenoxyethanol (CAS No. 122-99-6), 0.1-1% Imidazolidinyl Urea (CAS No. 39236-46-9), 0.1-1% Argania Spinosa Kernel Oil (CAS No. 223747-87-3), 0.1-1% Centella Asiatica Extract (CAS No. 84696-21-9), 0.1-1% Olea Europaea Leaf Extract (CAS No. 84012-27-1), 0.1-1% Aspalathus Linearis Extract (CAS No. 776295-36-4), 0.1-1% Triticum Vulgare Germ Oil (CAS No. 68917-73-7), 0.1-1% Parfum, 0.1-1% Nelumbo Nucifera Extract (CAS No. 85085-51-4), 0.1-1% Hamamelis Virginiana Extract (CAS No. 84696-19-5), 0.1-1% Sales, 0.1-1% Stevia Rebaudiana Extract (CAS No. 91722-21-3), < 0.1% Hordeum Vulgare Cera (CAS No. 85251-64-5), < 0.1% Methylparaben (CAS No. 99-76-3), < 0.1% alpha-Isomethyl lonone, < 0.1 % Linalool (CAS No. 78-70-6), < 0.1% Citric Acid (CAS No. 77-92-9), < 0.1% Ethylparaben (CAS No. 120-47-8), < 0.1% Propylparaben (CAS No. 94-13-3), < 0.1% Butylparaben (CAS No. 94-26-8), < 0.1% Hydroxycitronellal (CAS No. 107-75-5), < 0.1% Hydroxy Methylpentyl-3-cyclohexene-carboxaldehyde (CAS No. 31906-04-4), < 0.1% Hyaluronic Acid (CAS No. 9004-61-9), < 0.1% Citral (CAS No. 5392-40-5) < 0.1% Cinnamyl Alcohol (CAS No. 104-54-1), < 0.1% Amyl Cinnamal (CAS No. 122-40-7), < 0.1% Isobutylparaben (CAS No. 857259), < 0.1% Limonene (EINECS No. 5989-27-5) and < 0.1% Tocopherol (CAS No. 10191-41-0), and
c) where appropriate, the cosmetic preparation in the form of a cream contains, with respect to the total weight of the preparation, 6.019000% Steareth-10 (CAS No. 9005-00-9), 4.000000% Myristyl Myristate (CAS No. 3234-85-3), 2.990000% Cetearyl Isononanoate (CAS No. 84878-33-1), 1.989000% Glyceryl Stearate (CAS No. 31566-31-1), 1.521000% Cetearyl Alcohol (CAS No. 67762-27-0/8005-44-5), 1.400000% Glycoproteins (CAS No. 84604-16-0), 0.987000% Macadamia Ternifolia Seed Oil (CAS No. 128497-20-1/129811-19-4), 0.705000% Simmondsia Chinensis Oil (CAS No. 61789-91-1), 0.700000% Panthenol (CAS No. 81-13-0), 0.611000% Butyrospermum Parkii Butter (CAS No. 91080-23-8), 0.495380% Panthenyl Triacetate (CAS No. 94089-18-6), 0.481000% Dimethicone (CAS No. 9006-65-9/63148-62-9), 0.480823% Farnesol (CAS No. 4602-84-0), 0.480810% Farnesyl Acetate (CAS No. 29548-30-9), 0.427486% Zea Mays Germ Oil (CAS No. 8001-30-7), 0.420000% Propylene Glycol (CAS No. 57-55-6), 0.370000% Phenoxyethanol (CAS No. 122-99-6), 0.300000% Imidazolidinyl Urea (CAS No. 39236-46-9), 0.235000% Argania Spinosa Kernel Oil (CAS No. 223747-87-3), 0.210000% Centella Asiatica Extract (CAS No. 84696-21-9), 0.210000% Olea Europaea Leaf Extract (CAS No. 84012-27-1), 0.210000% Aspalathus Linearis Extract (CAS No. 776295-36-4), 0.182689% Triticum Vulgare Germ Oil (CAS No. 68917-73-7), 0.176213% Parfum, 0.157500% Nelumbo Nucifera Extract (CAS No. 85085-51-4), 0.157500% Hamamelis Virginiana Extract (CAS No. 84696-19-5), 0.150000% Sales, 0.105000% Stevia Rebaudiana Extract (CAS No. 91722-21-3), 0.094000% Hordeum Vulgare Cera (CAS No. 85251-64-5), 0.085000% Methylparaben (CAS No. 99-76-3), 0.025575% alpha-Isomethyl lonone, 0.023500% Linalool (CAS No. 78-70-6), 0.020000% Citric Acid (CAS No. 77-92-9), 0.020000% Ethylparaben (CAS No. 120-47-8), 0.015000% Propylparaben (CAS No. 94-13-3), 0.007500% Butylparaben (CAS No. 94-26-8), 0.006825% Hydroxycitronellal (CAS No. 107-75-5), 0.004500% Hydroxy Methylpentyl-3-cyclohexenecarboxaldehyde (CAS No. 31906-04-4), 0.004200% Hyaluronic Acid (CAS No. 9004-61-9), 0.004075% Citral (CAS No. 5392-40-5), 0.003000% Cinnamyl Alcohol (CAS No. 104-54-1), 0.002875% Amyl Cinnamal (CAS No. 122-40-7), 0.002500% Isobutylparaben (CAS No. 857259), 0.001750% Limonene (EINECS No. 5989-27-5), 0.000611% Tocopherol (CAS No. 10191-41-0) and water.

12. The preparation as claimed in one of claims 1 to 10, **characterized in that**
a) the cosmetic preparation is in the form of a body lotion,
b) where appropriate, the cosmetic preparation in the form of a lotion contains, with respect to the total weight of the preparation, ≥ 75% Aqua (CAS No. 7732-18-5), 1-5% Glycerin (CAS No. 56-81-5), 1-5% Zea Mays Germ Oil (CAS No. 8001-30-7), 1-5% Caprylic/Capric Triglyceride (CAS No. 73398-61-5/65381-09-1), 0.1-1% Polysorbate 85 (CAS No. 9005-70-3), 0.1-1% Macadamia Ternifolia Seed Oil (CAS No. 128497-20-1/129811-19-4), 0.1-1% Propylene Glycol (CAS No. 57-55-6), 0.1-1% Glycoproteins (CAS No. 84604-16-0), 0.1-1% Simmondsia Chinensis Oil (CAS No. 61789-91-1), 0.1-1% Triticum Vulgare Germ Oil (CAS No. 68917-73-7), 0.1-1% Saccharide Isomerate (CAS No. 100843-69-4), 0.1-1% Acrylates/Acrylamide Copolymer, 0.1-1% Petrolatum (8009-03-08), 0.1-1% Panthenol (CAS No. 81-13-0), 0.1-1% Calendula Officinalis Extract (CAS No. 84776-23-8), 0.1-1% Chamomilla Recutita Extract (CAS No.84082-60-0), 0.1-1% Butyrospermum Parkii Butter (CAS No. 91080-23-8), 0.1-1% Panthenyl Triacetate (CAS No. 94089-18-6), 0.1-1% Farnesyl Acetate (CAS No. 29548-30-9), 0.1-1% Farnesol (CAS No. 4602-84-0), 0.1-1% Imidazolidinyl Urea (CAS No. 39236-46-9), 0.1-1% Allantoin (CAS No. 97-59-6), 0.1-1% Carbomer (CAS No. 9007-20-9/9003-01-4), 0.1-1% Sales, 0.1-1% Argania Spinosa Kernel Oil (CAS No. 223747-87-3), 0.1-1% Olea Europaea Leaf Extract (CAS No. 84012-27-1), 0.1-1% Aspalathus Linearis Extract (CAS No. 776295-36-4), 0.1-1% Centella Asiatica Extract (CAS No. 84696-21-9), 0.1-1% Parfum, 0.1-1% Methylparaben (CAS No. 99-76-3), < 0.1% Nelumbo Nucifera Extract (CAS No. 85085-51-4), < 0.1% Hamamelis Virginiana Extract (CAS No. 84696-19-5), < 0.1% Hordeum Vulgare Cera (CAS No. 85251-64-5), < 0.1% Stevia Rebaudiana Extract (CAS No. 91722-21-3), < 0.1% Propylparaben (CAS No. 94-13-3), < 0.1% Citrus Grandis Oil (CAS No. 8016-20-4), < 0.1% Vanilla Planifolia Extract (CAS No. 84650-63-5), <0.1% Elettaria Cardamon Oil (CAS No. 8000-66-6), <0.1% Hexyl Cinnamal (CAS No. 101-86-0), < 0.1% Benzyl Benzoate (CAS No. 120-51-4), < 0.1% Linalool (CAS No. 78-70-6), < 0.1% Limonene, < 0.1% Hyaluronic Acid (CAS No. 9004-61-9), < 0.1% alpha-Isomethyl lonone, < 0.1% Tocopherol (CAS No. 10191-41-0), < 0.1% Citronellol (CAS No. 106-22-9) and < 0.1 % Retinyl Palmitate (CAS No. 79-81-2), and
c) where appropriate, the cosmetic preparation in the form of a lotion contains, with respect to the total weight of the preparation, 1.70000% Glycerin (CAS No. 56-81-5), 1.35232% Zea Mays Germ Oil (CAS No. 8001-30-7), 1.24196% Caprylic/Capric Triglyceride (CAS No. 73398-61-5/65381-09-1), 0.96000% Polysorbate 85 (CAS No. 9005-70-3), 0.83000% Macadamia Ternifolia Seed Oil (CAS No. 128497-20-1/129811-19-4), 0.80000% Propylene Glycol (CAS No. 57-55-6), 0.80000% Glycoproteins (CAS No. 84604-16-0), 0.65000% Simmondsia Chinensis Oil (CAS No. 61789-91-1), 0.59277% Triticum Vulgare Germ Oil (CAS No. 68917-73-7), 0.55000% Saccharide Isomerate (CAS No. 100843-69-4), 0.54000% Acrylates/Acrylamide Copolymer, 0.50000% Petrolatum (8009-03-08), 0.40000% Panthenol (CAS No. 81-13-0), 0.40000% Calendula Officinalis Extract (CAS No. 84776-23-8), 0.40000% Chamomilla Recutita Extract (CAS No. 84082-60-0), 0.39000% Butyrospermum Parkii Butter (CAS No. 91080-23-8), 0.31620% Panthenyl Triacetate (CAS No. 94089-18-6), 0.30690% Farnesyl Acetate (CAS No. 29548-30-9), 0.30690% Farnesol (CAS No. 4602-84-0), 0.30000% Imidazolidinyl Urea (CAS No. 39236-46-9), 0.30000% Allantoin (CAS No. 97-59-6), 0.25000% Carbomer (CAS No. 9007-20-9/9003-01-4), 0.15000% Sales, 0.15000% Argania Spinosa Kernel Oil (CAS No. 223747-87-3), 0.12000% Olea Europaea Leaf Extract (CAS No. 84012-27-1), 0.12000% Aspalathus Linearis Extract (CAS No. 776295-36-4), 0.12000% Centella Asiatica Extract (CAS No. 84696-21-9), 0.11642% Parfum, 0.11000% Methylparaben (CAS No. 99-76-3), 0.09000% Nelumbo Nucifera Extract (CAS No. 85085-51-4), 0.09000% Hamamelis Virginiana Extract (CAS No. 84696-19-5), 0.06000% Hordeum Vulgare Cera (CAS No. 85251-64-5), 0.06000% Stevia Rebaudiana Extract (CAS No. 91722-21-3), 0.03000% Propylparaben (CAS No. 94-13-3), 0.02000% Citrus Grandis Oil (CAS No. 8016-20-4), 0.01600% Vanilla Planifolia Extract (CAS No. 84650-63-5), 0.01500% Elettaria Cardamon Oil (CAS No. 8000-66-6), 0.00860% Hexyl Cinnamal (CAS No. 101-86-0), 0.00730% Benzyl Benzoate (CAS No. 120-51-4), 0.00600% Linalool (CAS No. 78-70-6), 0.00417% Limonene, 0.00240% Hyaluronic Acid (CAS No. 9004-61-9), 0.00200% alpha-Isomethyl lonone, 0.00239% Tocopherol (CAS No. 10191-41-0), 0.00180% Citronellol (CAS No. 106-22-9), 0.00056% Retinyl Palmitate (CAS No. 79-81-2) and water.

13. The preparation as claimed in one of claims 1 to 10, **characterized in that**
a) the cosmetic preparation is in the form of a hair tonic,
b) where appropriate, the cosmetic preparation in the form of a hair tonic contains, with respect to the total weight of the preparation, ≥ 50% Aqua (CAS No. 7732-18-5), 10-25% Mipa-Laureth Sulfate (CAS No. 83016-76-6/9062-04-8), 1-5% Mipa-Lauryl Sulfate (CAS No. 21142-28-9), 1-5% Cocoamidopropyl Betain (CAS No. 263-058-8), 1-5% Disodium Laureth Sulfosuccinate (CAS No. 39354-45-5), 1-5% PEG-12 Glyceryl Laurate (CAS No. 59070-56-3), 1-5% Disodium Cocoamphodiacetate (CAS No. 68650-39-5), 1-5% Laureth-13 (CAS No. 9002-92-0), 1-5% Glycoproteins (CAS No. 84604-16-0), 0.1-1% PEG-36 Castor Oil (CAS No. 61791-12-6), 0.1-1% Propylene Glycol (CAS No. 57-55-6), 0.1-1% Butylene Glycol (CAS No. 107-88-0), 0.1-1% Panthenol (CAS No. 81-13-0), 0.1-1% Linoleamide DEA (CAS No. 56863-02-6), 0.1-1% Farnesyl Acetate (CAS No. 29548-30-9), 0.1-1% Farnesol (CAS No. 4602-84-0), 0.1-1% Parfum, 0.1-1% PEG-10 Olive Glycerides, 0.1-1% Imidazolidinyl Urea (CAS No. 39236-46-9), 0.1-1% Hydrolyzed Milk Protein (CAS No. 92797-39-2/8049-98-7), 0.1-1% Olea Europaea Leaf Extract (CAS No. 84012-27-1), 0.1-1% Centella Asiatica Extract (CAS No. 84696-21-9), 0.1-1% Aspalathus Linearis Extract (CAS No. 776295-36-4), 0.1-1% Sales, 0.1-1% Nelumbo Nucifera Extract (CAS No. 85085-51-4), 0.1-1% Hamamelis Virginiana Extract (CAS No. 84696-19-5), 0.1-1% Methylparaben (CAS No. 99-76-3), 0.1-1% Panicum Miliaceum Extract (CAS No. 90082-36-3), 0.1-1% Salvia Officinalis Extract (CAS No. 84082-79-1), 0.1-1% Adenosine Triphosphate (CAS No. 56-65-5), 0.1-1% Betula Alba Leaf Extract (CAS No. 84012-15-7), 0.1-1% Polyquaternium-10 (CAS No. 81859-24-7/53568-66-4), 0.1-1% Chamomilla Recutita Extract (CAS No. 84082-60-0), 0.1-1% Niacinamide (CAS No. 98-92-0), < 0.1 % Camelia Sinensis Extract (CAS No. 84650-60-2), < 0.1% Tilia Cordata Flower Extract (CAS No. 84929-52-2), < 0.1% Urtica Dioica Extract (CAS No. 84012-40-8), < 0.1% Panthenyl Triacetate (CAS No. 94089-18-6), < 0.1% Humulus Lupulus Extract (CAS No. 8060-28-4), < 0.1% Cinchona Pubescens Extract (CAS No. 84776-28-3), < 0.1% Equisetum Arvense Extract (CAS No. 71011-23-9), < 0.1% Stevia Rebaudiana Extract (CAS No. 91722-21-3), < 0.1% Citrus Grandis Oil (CAS No. 8016-20-4), < 0.1% Vanilla Planifolia Extract (CAS No. 84650-63-5), < 0.1% Elettaria Cardamon Oil (CAS No. 8000-66-6), < 0.1% Propylparaben (CAS No. 94-13-3), < 0.1% Hexyl Cinnamal (CAS No. 101-86-0), < 0.1% Benzyl Benzoate (CAS No. 120-51-4), < 0.1% Linalool (CAS No. 78-70-6), < 0.1% Limonene, < 0.1 % Niacin (CAS No. 59-67-6), < 0.1 % Hyaluronic Acid (CAS No. 9004-61-9) and < 0.1% Biotin (CAS No. 58-85-5), and
c) where appropriate, the cosmetic preparation in the form of a hair tonic contains, with respect to the total weight of the preparation, 10.00000% Mipa-Laureth Sulfate (CAS No. 83016-76-6/9062-04-8), 3.15000% Mipa-Lauryl Sulfate (CAS No. 21142-28-9), 1.60000% Cocoamidopropyl Betain (CAS No. 263-058-8), 1.50000% Disodium Laureth Sulfosuccinate (CAS No. 39354-45-5), 1.20000% PEG-12 Glyceryl Laurate (CAS No. 59070-56-3), 1.20000% Disodium Cocoamphodiacetate (CAS No. 68650-39-5), 1.20000% Laureth-13 (CAS No. 9002-92-0), 1.00000% Glycoproteins (CAS No. 84604-16-0), 0.90000% PEG-36 Castor Oil (CAS No. 61791-12-6), 0.86000% Propylene Glycol (CAS No. 57-55-6), 0.60000% Butylene Glycol (CAS No. 107-88-0), 0.54990% Panthenol (CAS No. 81-13-0), 0.45000% Linoleamide DEA (CAS No. 56863-02-6), 0.45000% Farnesyl Acetate (CAS No. 29548-30-9), 0.36000% Farnesol (CAS No. 4602-84-0), 0.34927% Parfum, 0.30000% PEG-10 Olive Glycerides, 0.30000% Imidazolidinyl Urea (CAS No. 39236-46-9), 0.20000% Hydrolyzed Milk Protein (CAS No. 92797-39-2/8049-98-7), 0.15000% Olea Europaea Leaf Extract (CAS No. 84012-27-1), 0.15000% Centella Asiatica Extract (CAS No. 84696-21-9), 0.15000% Aspalathus Linearis Extract (CAS No. 776295-36-4), 0.15000% Sales, 0.11250% Nelumbo Nucifera Extract (CAS No. 85085-51-4), 0.11250% Hamamelis Virginiana Extract (CAS No. 84696-19-5), 0.11000% Methylparaben (CAS No. 99-76-3), 0.10000% Panicum Miliaceum Extract (CAS No. 90082-36-3), 0.10000% Salvia Officinalis Extract (CAS No. 84082-79-1), 0.10000% Adenosine Triphosphate (CAS No. 56-65-5), 0.10000% Betula Alba Leaf Extract (CAS No. 84012-15-7), 0.10000% Polyquaternium-10 (CAS No. 81859-24-7/53568-66-4), 0.10000% Chamomilla Recutita Extract (CAS No. 84082-60-0), 0.10000% Niacinamide (CAS No. 98-92-0), 0.09000% Camelia Sinensis Extract (CAS No. 84650-60-2), 0.09000% Tilia Cordata Flower Extract (CAS No. 84929-52-2), 0.09000% Urtica Dioica Extract (CAS No. 84012-40-8), 0.09000% Panthenyl Triacetate (CAS No. 94089-18-6), 0.09000% Humulus Lupulus Extract (CAS No. 8060-28-4), 0.09000% Cinchona Pubescens Extract (CAS No. 84776-28-3), 0.09000% Equisetum Arvense Extract (CAS No. 71011-23-9), 0.07500% Stevia Rebaudiana Extract (CAS No. 91722-21-3), 0.06000% Citrus Grandis Oil (CAS No. 8016-20-4), 0.04800% Vanilla Planifolia Extract (CAS No. 84650-63-5), 0.04500% Elettaria Cardamon Oil (CAS No. 8000-66-6), 0.03000% Propylparaben (CAS No. 94-13-3), 0.02580% Hexyl Cinnamal (CAS No. 101-86-0), 0.02190% Benzyl Benzoate (CAS No. 120-51-4), 0.01801% Linalool (CAS No. 78-70-6), 0.01252% Limonene, 0.01000% Niacin (CAS No. 59-67-6), 0.00300% Hyaluronic Acid (CAS No. 9004-61-9), 0.00010% Biotin (CAS No. 58-85-5) and water.

14. The preparation as claimed in one of claims 1 to 10, **characterized in that**
a) the cosmetic preparation is in the form of a shower shampoo,
b) where appropriate, the cosmetic preparation in the form of a shower shampoo contains, with respect to the total weight of the preparation, ≥ 50% Aqua (CAS No. 7732-18-5), 10-25% Mipa-Laureth Sulfate (CAS No. 83016-76-6/9062-04-8), 1-5% Mipa-Lauryl Sulfate (CAS No. 21142-28-9), 1-5% Cocoamidopropyl Betain (CAS No. 263-058-8), 1-5% Disodium Laureth Sulfosuccinate (CAS No. 39354-45-5), 1-5% Laureth-13 (CAS No. 9002-92-0), 1-5% Disodium Cocoamphodiacetate (CAS No. 68650-39-5), 0.1-1% Sodium Chloride (CAS No. 7647-14-5), 0.1-1% Propylene Glycol (CAS No. 57-55-6), 0.1-1% Glycoproteins (CAS No. 84604-16-0), 0.1-1% Linoleamide DEA (CAS No. 56863-02-6), 0.1-1% Panthenol (CAS No. 81-13-0), 0.1-1% Polyquaternium-10 (CAS No. 81859-24-7/53568-66-4), 0.1-1% PEG-10 Olive Glycerides, 0.1-1% Imidazolidinyl Urea (CAS No. 39236-46-9), 0.1-1% Panicum Miliaceum Extract (CAS No. 90082-36-3), 0.1-1% Chamomilla Recutita Extract (CAS No. 84082-60-0), 0.1-1% Salvia Officinalis Extract (CAS No. 84082-79-1), 0.1-1% Betula Alba Leaf Extract (CAS No. 84012-15-7), 0.1-1% Parfum, 0.1-1 % Tilia cordata Flower Extract (CAS No. 84929-52-2), 0.1-1% Urtica Dioica Extract (CAS No. 84012-40-8), 0.1-1% Camelia Sinensis Extract (CAS No. 84650-60-2), 0.1-1% Equisetum Arvense Extract (CAS No. 71011-23-9), 0.1-1% Humulus Lupulus Extract (CAS No. 8060-28-4), 0.1-1% Cinchona Extract (CAS No. 84776-28-3), 0.1-1% Sales, 0.1-1% PEG-12 Glyceryl Laurate (CAS No. 59070-56-3), 0.1-1% Methylparaben (CAS No. 99-76-3), < 0.1% PEG-36 Castor Oil (CAS No. 61791-12-6), < 0.1% Centella Asiatica Extract (CAS No. 84696-21-9), < 0.1% Olea Europaea Leaf Extract (CAS No. 84012-27-1), < 0.1% Aspalathus Linearis Extract (CAS No. 776295-36-4), < 0.1% Hamamelis Virginiana Extract (CAS No. 84696-19-5), < 0.1% Nelumbo Nucifera Extract (CAS No. 85085-51-4), < 0.1% Farnesyl Acetate (CAS No. 29548-30-9), < 0.1% Citrus Grandis Oil (CAS No. 8016-20-4), < 0.1% Stevia Rebaudiana Extract (CAS No. 91722-21-3), <0.1% Farnesol (CAS No. 4602-84-0), < 0.1 % Vanilla Planifolia Extract (CAS No. 84650-63-5), < 0.1% Elettaria Cardamon Oil (CAS No. 8000-66-6), < 0.1% Propylparaben (CAS No. 94-13-3), < 0.1% Niacin (CAS No. 59-67-6), <0.1% Hexyl Cinnamal (CAS No. 101-86-0), < 0.1% Benzyl Benzoate (CAS No. 120-51-4), < 0.1% Linalool (CAS No. 78-70-6) and < 0.1% Hyaluronic Acid (CAS No. 9004-61-9), and
c) where appropriate, the cosmetic preparation in the form of a shower shampoo contains, with respect to the total weight of the preparation, 10.00000% Mipa-Laureth Sulfate (CAS No. 83016-76-6/9062-04-8), 3.15000% Mipa-Lauryl Sulfate (CAS No. 21142-28-9), 1.60000% Cocoamidopropyl Betain (CAS No. 263-058-8), 1.50000% Disodium Laureth Sulfosuccinate (CAS No. 39354-45-5), 1.20000% Laureth-13 (CAS No. 9002-92-0), 1.20000% Disodium Cocoamphodiacetate (CAS No. 68650-39-5), 0.90000% Sodium Chloride (CAS No. 7647-14-5), 0.71000% Propylene Glycol (CAS No. 57-55-6), 0.50000% Glycoproteins (CAS No. 84604-16-0), 0.45000% Linoleamide DEA (CAS No. 56863-02-6), 0.37475% Panthenol (CAS No. 81-13-0), 0.30000% Polyquaternium-10 (CAS No. 81859-24-7/53568-66-4), 0.30000% PEG-10 Olive Glycerides, 0.30000% Imidazolidinyl Urea (CAS No. 39236-46-9), 0.25000% Panicum Miliaceum Extract (CAS No. 90082-36-3), 0.25000% Chamomilla Recutita Extract (CAS No. 84082-60-0), 0.25000% Salvia Officinalis Extract (CAS No. 84082-79-1), 0.25000% Betula Alba Leaf Extract (CAS No. 84012-15-7), 0.23285% Parfum, 0.22500% Tilia Cordata Flower Extract (CAS No. 84929-52-2), 0.22500% Urtica Dioica Extract (CAS No. 84012-40-8), 0.22500% Camelia Sinensis Extract (CAS No. 84650-60-2), 0.22500% Equisetum Arvense Extract (CAS No. 71011-23-9), 0.22500% Humulus Lupulus Extract (CAS No. 8060-28-4), 0.22500% Cinchona Extract (CAS No. 84776-28-3), 0.15000% Sales, 0.12000% PEG-12 Glyceryl Laurate (CAS No. 59070-56-3), 0.11000% Methylparaben (CAS No. 99-76-3), 0.09000% PEG-36 Castor Oil (CAS No. 61791-12-6), 0.075001% Centella Asiatica Extract (CAS No. 84696-21-9), 0.07500% Olea Europaea Leaf Extract (CAS No. 84012-27-1), 0.07500% Aspalathus Linearis Extract (CAS No. 776295-36-4), 0.05625% Hamamelis Virginiana Extract (CAS No. 84696-19-5), 0.05625% Nelumbo Nucifera Extract (CAS No. 85085-51-4), 0.04500% Farnesyl Acetate (CAS No. 29548-30-9), 0.04000% Citrus Grandis Oil (CAS No. 8016-20-4), 0.03750% Stevia Rebaudiana Extract (CAS No. 91722-21-3), 0.03600% Farnesol (CAS No. 4602-84-0), 0.03200% Vanilla Planifolia Extract (CAS No. 84650-63-5), 0.03000% Elettaria Cardamon Oil (CAS No. 8000-66-6), 0.03000% Propylparaben (CAS No. 94-13-3), 0.02500% Niacin (CAS No. 59-67-6), 0.01720% Hexyl Cinnamal (CAS No. 101-86-0), 0.01460% Benzyl Benzoate (CAS No. 120-51-4), 0.01201% Linalool (CAS No. 78-70-6), 0.00150% Hyaluronic Acid (CAS No. 9004-61-9) and water.

15. Use of a sodium-chloride-iron-iodine thermal water with an electrolyte concentration of at least 25 g/kg, preferably at least 30 g/kg, and an iron content of at least 9 mg/kg, for the manufacture of a cosmetic preparation for skin care as claimed in one of claims 1 to 14.

## Revendications

1. Préparation cosmétique destinée aux soins de la peau, **caractérisée en ce qu'**elle contient, par rapport au poids total de la préparation, outre les agents auxiliaires et additifs traditionnellement utilisés en cosmétique,
(a) au moins 2 % d'un composant hydrophile contenant Aqua (CAS-n° 7732-18-5), Glycoproteins (CAS-n° 84604-16-0), Panthenol (CAS-n° 81-13-0), Propylene Glycol (CAS-n° 57-55-6), Olea Europaea Leaf Extract (CAS-n° 84012-27-1), Aspalathus Linearis Extract (CAS-n° 776295-36-4), Centella Asiatica Extract (CAS-n° 84696-21-9), Hamamelis Virginiana Extract (CAS-n° 84696-19-5), Nelumbo Nucifera Extract (CAS-n° 85085-51-4), Stevia Rebaudiana Extract (CAS-n° 91722-21-3) et Hyaluronic Acid (CAS-n° 9004-61-9),
(b) au moins 10 % d'eau thermale de type sodium - chlorure - fer - iode ayant une teneur globale en électrolytes supérieure ou égale à 25 g/kg, de préférence supérieure ou égale à 30 g/kg, et une teneur en fer supérieure ou égale à 9 mg/kg
ainsi qu'éventuellement
(c) jusqu'à 15 % d'un composant lipophile contenant un ou plusieurs ingrédients choisis dans le groupe constitué de Macadamia Ternifolia Seed Oil (CAS-n° 128497-20-1/129811-19-4), Simmondsia Chinensis Seed Oil (CAS-n° 61789-91-1), Butyrospermum Parkii Butter (CAS-n° 91080-23-8), Panthenyl Triacetate (CAS-n° 94089-18-6), Farnesyl Acetate (CAS-n° 29548-30-9), Farnesol (CAS-n° 4602-84-0), Zea Mays Germ Oil (CAS-n° 8001-30-7), Argania Spinosa Kernel Oil (CAS-n° 223747-87-3), Triticum Vulgare Germ Oil (CAS-n° 68917-73-7), Hordeum Vulgare Cera (CAS-n° 85251-64-5), Tocopherol (CAS-n° 10191-41-0) et Retinyl Palmitate (CAS-n° 79-81-2).

2. Préparation selon la revendication 1, **caractérisée en ce que** ladite eau thermale de type sodium - chlorure - fer - iode présente une teneur globale en électrolytes supérieure ou égale à 37 g/kg.

3. Préparation selon les revendications 1 ou 2, **caractérisée en ce qu'**elle contient 10 à 80 % en poids d'eau thermale de type sodium - chlorure - fer - iode.

4. Préparation selon l'une des revendications 1 à 3, **caractérisée en ce que** ledit composant hydrophile contient, par rapport au poids dudit composant, 25 à 50 % Aqua (CAS-n° 7732-18-5), 10 à 25 % Glycoproteins (CAS-n° 84604-16-0), 10 à 25 % Panthenol (CAS-n° 81-13-0), 5 à 10 % Propylene Glycol (CAS-n° 57-55-6), 1 à 5 % Olea Europaea Leaf Extract (CAS-n° 84012-27-1), 1 à 5 % Aspalathus Linearis Extract (CAS-n° 776295-36-4), 1 à 5 % Centella Asiatica Extract (CAS-n° 84696-21-9), 1 à 5 % Hamamelis Virginiana Extract (CAS-n° 84696-19-5), 1 à 5 % Nelumbo Nucifera Extract (CAS-n° 85085-51-4), 1 à 5 % Stevia Rebaudiana Extract (CAS-n° 91722-21-3) et < 0,1% Hyaluronic Acid (CAS-n° 9004-61-9).

5. Préparation selon la revendication 4, **caractérisée en ce que** ledit composant hydrophile contient, par rapport au poids dudit composant, 48,9400 % Aqua (CAS-n° 7732-18-5), 20,0000 % Glycoproteins (CAS-n° 84604-16-0), 10,0000 % Panthenol (CAS-n° 81-13-0), 6,0000 % Propylene Glycol (CAS-n° 57-55-6), 3,0000 % Olea Europaea Leaf Extract (CAS-n° 84012-27-1), 3,0000 % Aspalathus Linearis Extract (CAS-n° 776295-36-4), 3,0000 % Centella Asiatica Extract (CAS-n° 84696-21-9), 2,2500 % Hamamelis Virginiana Extract (CAS-n° 84696-19-5), 2,2500 % Nelumbo Nucifera Extract (CAS-n° 85085-51-4), 1,5000 % Stevia Rebaudiana Extract (CAS-n° 91722-21-3) et 0,0600 % Hyaluronic Acid (CAS-n° 9004-61-9).

6. Préparation selon l'une des revendications 1 à 5, **caractérisée en ce qu'**elle contient ledit composant hydrophile dans une quantité comprise entre 2 et 10 %, par rapport au poids de ladite préparation.

7. Préparation selon l'une des revendications 1 à 6, **caractérisée en ce que** ledit composant lipophile contient, par rapport au poids dudit composant, 10 à 25 % Macadamia Ternifolia Seed Oil (CAS-n° 128497-20-1/129811-19-4), 10 à 25 % Simmondsia Chinensis Seed Oil (CAS-n° 61789-91-1), 10 à 25 % Butyrospermum Parkii Butter (CAS-n° 91080-23-8), 10 à 25 % Panthenyl Triacetate (CAS-n° 94089-18-6), 10 à 25 % Farnesyl Acetate (CAS-n° 29548-30-9), 10 à 25 % Farnesol (CAS-n° 4602-84-0), 5 à 10 % Zea Mays Germ Oil (CAS-n° 8001-30-7), 5 à 10 % Argania Spinosa Kernel Oil (CAS-n° 223747-87-3), 1 à 5 % Triticum Vulgare Germ Oil (CAS-n° 6891773-7), 1 à 5 % Hordeum Vulgare Cera (CAS-n° 85251-64-5), < 0,1 % Tocopherol (CAS-n° 10191-41-0) et < 0,1% Retinyl Palmitate (CAS-n° 79-81-2).

8. Préparation selon la revendication 7, **caractérisée en ce que** ledit composant lipophile contient, par rapport au poids dudit composant, 21,0000 % Macadamia Ternifolia Seed Oil (CAS-n° 128497-20-1/129811-19-4), 15,0000 % Simmondsia Chinensis Seed Oil (CAS-n° 61789-91-1), 13,0000 % Butyrospermum Parkii Butter (CAS-n° 91080-23-8), 10,5400 % Panthenyl Triacetate (CAS-n° 94089-18-6), 10,2300 % Farnesyl Acetate (CAS-n° 29548-30-9), 10,2300 % Farnesol (CAS-n° 4602-84-0), 9,0955 % Zea Mays Germ Oil (CAS-n° 8001-30-7), 5,0000 % Argania spinosa Kernel Oil (CAS-n° 223747-87-3), 3,8870 % Triticum Vulgare Germ Oil (CAS-n° 68917-73-7), 2,0000 % Hordeum Vulgare Cera (CAS-n° 85251-64-5), 0,0130 % Tocopherol (CAS-n° 10191-41-0) et 0,0046 % Retinyl Palmitate (CAS-n° 79-81-2).

9. Préparation selon l'une des revendications 1 à 8, **caractérisée en ce qu'**elle contient au moins un additif choisi dans le groupe constitué d'Allantoin (CAS-n° 97-59-6), Adenosintriphosphat (CAS-n° 56-65-5), Betula Alba Leaf Extract (CAS-n° 84012-15-7), Biotin (CAS-n° 58-85-5), Citrus Grandis Oil (CAS-n° 8016-20-4), Cinchona Extract (CAS-n° 84776-28-3), Camelia Sinensis Extract (CAS-n° 84650-60-2), Chamomilla Recutita Extract (CAS-n° 84082-60-0), Equisetum Arvense Extract (CAS-n° 71011-23-9), Humulus Lupulus Extract (CAS-n° 8060-28-4), Niacinamid (CAS-n° 98-92-0), Niacin (CAS-n° 59-67-6), Panicum Miliaceum Extract (CAS-n° 90082-36-3), Salvia Officinalis Extract (CAS-n° 84082-79-1), Tilia Cordata Flower Extract (CAS-n° 84929-52-2), Urtica Dioica Extract (CAS-n° 84012-40-8) et Vanille Planifolia Extract (CAS-n° 84650-63-5).

10. Préparation selon l'une des revendications 1 à 9, **caractérisée en ce qu'**elle contient un ou plusieurs agents auxiliaires traditionnels lesquels sont choisis parmi les émulsifiants, les co-émulsifiants, les solvants, les agents de solubilisation, les conservateurs, les antioxydants, les agents complexants, les agents humectants, les agents filmogènes, les gélifiants, les agents régulateurs de texture, les tampons, les agents stabilisateurs, les agents antistatiques, les filtres UV, les colorants, les agents désodorisants, les parfums, les agents masquants, les agents tensioactifs, les agents régulateurs de mousse et les agents stabilisateurs de mousse.

11. Préparation cosmétique selon l'une des revendications 1 à 10, **caractérisée en ce que**
a) ladite préparation cosmétique se présente sous forme d'une crème,
b) ladite préparation cosmétique, laquelle se présente sous forme d'une crème, contenant le cas échéant, par rapport au poids total de ladite préparation, ≥ 50% Aqua (CAS-n° 7732-18-5), 5 à 10 % Steareth-10 (CAS-n° 9005-00-9), 1 à 5 % Myristyl Myristate (CAS-n° 3234-85-3), 1 à 5 % Cetearyl Isononanoate (CAS-n° 84878-33-1), 1 à 5 % Glyceryl Stearate (CAS-n° 31566-31-1), 1 à 5 % Cetearyl Alcohol (CAS-n° 67762-27-0/8005-44-5), 1 à 5 % Glycoproteins (CAS-n° 84604-16-0), 0,1 à 1 % Macadamia Ternifolia Seed Oil (CAS-n° 128497-20-1/129811-19-4), 0,1 à 1 % Simmondsia Chinensis Oil (CAS-n° 61789-91-1), 0,1 à 1 % Panthenol (CAS-n° 81-13-0), 0,1 à 1 % Butyrospermum Parkii Butter (CAS-n° 91080-23-8), 0,1 à 1% Panthenyl Triacetate (CAS-n° 94089-18-6), 0,1 à 1 % Dimethicone (CAS-n° 9006-65-9/63148-62-9), 0,1 à 1 % Farnesol (CAS-n° 4602-84-0), 0,1 à 1 % Farnesyl Acetate (CAS-n° 29548-30-9), 0,1 à 1 % Zea Mays Germ Oil (CAS-n° 8001-30-7), 0,1 à 1 % Propylene Glycol (CAS-n° 57-55-6), 0,1 à 1 % Phenoxyethanol (CAS-n° 122-99-6), 0,1 à 1 % Imidazolidinyl Urea (CAS-n° 39236-46-9), 0,1 à 1 % Argania Spinosa Kernel Oil (CAS-n° 223747-87-3), 0,1 à 1 % Centella Asiatica Extract (CAS-n° 84696-21-9), 0,1 à 1 % Olea Europaea Leaf Extract (CAS-n° 84012-27-1), 0,1 à 1 % Aspalathus Linearis Extract (CAS-n° 776295-36-4), 0,1 à 1 % Triticum Vulgare Germ Oil (CAS-n° 68917-73-7), 0,1 à 1 % Parfum, 0,1 à 1 % Nelumbo Nucifera Extract (CAS-n° 85085-51-4), 0,1 à 1 % Hamamelis Virginiana Extract (CAS-n° 84696-19-5), 0,1 à 1 % Sales, 0,1 à 1 % Stevia Rebaudiana Extract (CAS-n° 91722-21-3), < 0,1 % Hordeum vulgare Cera (CAS-n° 85251-64-5), < 0,1 % Methylparaben (CAS-n° 99-76-3), < 0,1 % alpha-Isomethyl lonone, < 0,1% Linalool (CAS-n° 78-70-6), < 0,1 % Citric Acid (CAS-n° 77-92-9), < 0,1 % Ethylparaben (CAS-n° 120-47-8), < 0,1 % Propylparaben (CAS-n° 94-13-3), < 0,1 % Butylparaben (CAS-n° 94-26-8), < 0,1 % Hydroxycitronellal (CAS-n° 107-75-5), < 0,1 % Hydroxy Methylpentyl-3-cyclohexene-carboxaldehyde (CAS-n° 31906-04-4), < 0,1 % Hyaluronic Acid (CAS-n° 9004-61-9), < 0,1 % Citral (CAS-n° 5392-40-5) < 0,1% Cinnamyl Alcohol (CAS-n° 104-54-1), < 0,1 % Amyl Cinnamal (CAS-n° 122-40-7), < 0,1 % Isobutylparaben (CAS-n° 857259), < 0,1 % Limonene (EINECS-n° 5989-27-5) et < 0,1 % Tocopherol (CAS-n° 10191-41-0) et,
c) ladite préparation cosmétique, laquelle se présente sous forme d'une crème, contenant le cas échéant, par rapport au poids total de ladite préparation, 6,019000 % Steareth-10 (CAS-n° 9005-00-9), 4,000000 % Myristyl Myristate (CAS-n° 3234-85-3), 2,990000 % Cetearyl Isononanoate (CAS-n° 84878-33-1), 1,989000 % Glyceryl Stearate (CAS-n° 31566-31-1), 1,521000 % Cetearyl Alcohol (CAS-n° 67762-27-0/8005-44-5), 1,400000 % Glycoproteins (CAS-n° 84604-16-0), 0,987000 % Macadamia Ternifolia Seed Oil (CAS-n° 128497-20-1/129811-19-4), 0,705000 % Simmondsia Chinensis Oil (CAS-n° 61789-91-1), 0,700000 % Panthenol (CAS-n° 81-13-0), 0,611000 % Butyrospermum Parkii Butter (CAS-n° 91080-23-8), 0,495380 % Panthenyl Triacetate (CAS-n° 94089-18-6), 0,481000 % Dimethicone (CAS-n° 9006-65-9/63148-62-9), 0,480823 % Farnesol (CAS-n° 4602-84-0), 0,480810 % Farnesyl Acetate (CAS-n° 29548-30-9), 0,427486 % Zea Mays Germ Oil (CAS-n° 8001-30-7), 0,420000 % Propylene Glycol (CAS-n° 57-55-6), 0,370000 % Phenoxyethanol (CAS-n° 122-99-6), 0,300000 % Imidazolidinyl Urea (CAS-n° 39236-46-9), 0,235000 % Argania Spinosa Kernel Oil (CAS-n° 223747-87-3), 0,210000 % Centella Asiatica Extract (CAS-n° 84696-21-9), 0,210000 % Olea Europaea Leaf Extract (CAS-n° 84012-27-1), 0,210000 % Aspalathus Linearis Extract (CAS-n° 776295-36-4), 0,182689 % Triticum Vulgare Germ Oil (CAS-n° 68917-73-7), 0,176213 % Parfum, 0,157500 % Nelumba Nucifera Extract (CAS-n° 85085-51-4), 0,157500 % Hamamelis Virginiana Extract (CAS-n° 84696-19-5), 0,150000 % Sales, 0,105000 % Stevia Rebaudiana Extract (CAS-n° 91722-21-3), 0,094000 % Hordeum Vulgare Cera (CAS-n° 85251-64-5), 0,085000 % Methylparaben (CAS-n° 99-76-3), 0,025575 % alpha-Isomethyl lonone, 0,023500% Linalool (CAS-n° 78-70-6), 0,020000 % Citric Acid (CAS-n° 77-92-9), 0,020000 % Ethylparaben (CAS-n° 120-47-8), 0,015000 % Propylparaben (CAS-n° 94-13-3), 0,007500 % Butylparaben (CAS-n° 94-26-8), 0,006825 % Hydroxycitronellal (CAS-n° 107-75-5), 0,004500 % Hydroxy Methylpentyl-3-cyclohexene-carboxaldehyde (CAS-n° 31906-04-4), 0,004200 % Hyaluronic Acid (CAS-n° 9004-61-9), 0,004075 % Citral (CAS-n° 5392-40-5), 0,003000% Cinnamyl Alcohol (CAS-n° 104-54-1), 0,002875 % Amyl Cinnamal (CAS-n° 122-40-7), 0,002500 % Isobutylparaben (CAS-n° 857259), 0,001750 % Limonene (EINECS-n°. 5989-27-5), 0,000611 % Tocopherol (CAS-n° 10191-41-0) et de l'eau.

12. Préparation selon l'une des revendications 1 à 10, **caractérisée en ce que**
a) ladite préparation cosmétique se présente sous forme d'une lotion pour le corps,
b) ladite préparation cosmétique, laquelle se présente sous forme d'une lotion, contenant le cas échéant, par rapport au poids total de ladite préparation, ≥ 75% Aqua (CAS-n° 7732-18-5), 1 à 5 % Glycerin (CAS-n° 56-81-5), 1 à 5 % Zea Mays Germ Oil (CAS-n° 8001-30-7), 1 à 5 % Caprylic/Capric Triglyceride (CAS-n° 73398-61-5/65381-09-1), 0,1 à 1 % Polysorbate 85 (CAS-n° 9005-70-3), 0,1 à 1 % Macadamia Ternifolia Seed Oil (CAS-n° 128497-20-1/129811-19-4), 0,1 à 1 % Propylene Glycol (CAS-n° 57-55-6), 0,1 à 1 % Glycoproteins (CAS-n° 84604-16-0), 0,1 à 1 % Simmondsia Chinensis Oil (CAS-n° 61789-91-1), 0,1 à 1 % Triticum Vulgare Germ Oil (CAS-n° 68917-73-7), 0,1 à 1 % Saccharide Isomerate (CAS-n° 100843-69-4), 0,1 à 1% Acrylates/Acrylamide Copolymer, 0,1 à 1 % Petrolatum (8009-03-08), 0,1 à 1 % Panthenol (CAS-n° 81-13-0), 0,1 à 1 % Calendula Officinalis Extract (CAS-n° 84776-23-8), 0,1 à 1 % Chamomilla Recutita Extract (CAS-n° 84082-60-0), 0,1 à 1 % Butyrospermum Parkii Butter (CAS-n° 91080-23-8), 0,1 à 1 % Panthenyl Triacetate (CAS-n° 94089-18-6), 0,1 à 1 % Farnesyl Acetate (CAS-n° 29548-30-9), 0,1 à 1 % Farnesol (CAS-n° 4602-84-0), 0,1 à 1 % Imidazolidinyl Urea (CAS-n° 39236-46-9), 0,1 à 1 % Allantoin (CAS-n° 97-59-6), 0,1 à 1 % Carbomer (CAS-n° 9007-20-9/9003-01-4), 0,1 à 1 % Sales, 0,1 à 1 % Argania spinosa Kernel Oil (CAS-n° 223747-87-3), 0,1 à 1 % Olea Europaea Leaf Extract (CAS-n° 84012-27-1), 0,1 à 1 % Aspalathus Linearis Extract (CAS-n° 776295-36-4), 0,1 à 1 % Centella Asiatica Extract (CAS-n° 84696-21-9), 0,1 à 1 % Parfum, 0,1 à 1 % Methylparaben (CAS-n° 99-76-3), < 0,1 % Nelumbo Nucifera Extract (CAS-n° 85085-51-4), < 0,1 % Hamamelis Virginiana Extract (CAS-n° 84696-19-5), < 0,1 % Hordeum Vulgare Cera (CAS-n° 85251-64-5), < 0,1 % Stevia Rebaudiana Extract (CAS-n° 91722-21-3), < 0,1 % Propylparaben (CAS-n° 94-13-3), < 0,1 % Citrus Grandis Oil (CAS-n° 8016-20-4), < 0,1 % Vanilla Planifolia Extract (CAS-n° 84650-63-5), < 0,1 % Elettaria Cardamon Oil (CAS-n° 8000-66-6), < 0,1 % Hexyl Cinnamal (CAS-n° 101-86-0), < 0,1 % Benzyl Benzoate (CAS-n° 120-51-4), < 0,1 % Linalool (CAS-n° 78-70-6), < 0,1 % Limonene, < 0,1 % Hyaluronic Acid (CAS-n° 9004-61-9), < 0,1 % alpha-Isomethyl lonone, < 0,1 % Tocopherol (CAS-n° 10191-41-0), < 0,1 % Citronellol (CAS-n° 106-22-9) et < 0,1 % Retinyl Palmitate (CAS-n° 79-81-2) et
c) ladite préparation cosmétique, laquelle se présente sous forme d'une lotion, contenant le cas échéant, par rapport au poids total de ladite préparation, 1,70000% Glycerin (CAS-n° 56-81-5), 1,35232 % Zea Mays Germ Oil (CAS-n° 8001-30-7), 1,24196 % Caprylic/Capric Triglyceride (CAS-n° 73398-61-5/65381-09-1), 0,96000 % Polysorbate 85 (CAS-n° 9005-70-3), 0,83000 % Macadamia Ternifolia Seed Oil (CAS-n° 128497-20-1/129811-19-4), 0,80000 % Propylene Glycol (CAS-n° 57-55-6), 0,80000 % Glycoproteins (CAS-n° 84604-16-0), 0,65000 % Simmondsia Chinensis Oil (CAS-n° 61789-91-1), 0,59277 % Triticum Vulgare Germ Oil (CAS-n° 68917-73-7), 0,55000 % Saccharide isomerate (CAS-n° 100843-69-4), 0,54000 % Acrylates/Acrylamide Copolymer, 0,50000 % Petrolatum (8009-03-08), 0,40000 % Panthenol (CAS-n° 81-13-0), 0,40000 % Calendula Officinalis Extract (CAS-n° 84776-23-8), 0,40000 % Chamomilla Recutita Extract (CAS-n° 84082-60-0), 0,39000 % Butyrospermum Parkii Butter (CAS-n° 91080-23-8), 0,31620 % Panthenyl Triacetate (CAS-n° 94089-18-6), 0,30690 % Farnesyl Acetate (CAS-n° 29548-30-9), 0,30690 % Farnesol (CAS-n° 4602-84-0), 0,30000 % Imidazolidinyl Urea (CAS-n° 39236-46-9), 0,30000 % Allantoin (CAS-n° 97-59-6), 0,25000 % Carbomer (CAS-n° 9007-20-919003-01-4), 0,15000 % Sales, 0,15000 % Argania Spinosa Kernel Oil (CAS-n° 223747-87-3), 0,12000 % Olea Europaea Leaf Extract (CAS-n° 84012-27-1), 0,12000 % Aspalathus Linearis Extract (CAS-n° 776295-36-4), 0,12000 % Centella Asiatica Extract (CAS-n° 84696-21-9), 0,11642 % Parfum, 0,11000 % Methylparaben (CAS-n° 99-76-3), 0,09000 % Nelumbo Nucifera Extract (CAS-n° 85085-51-4), 0,09000 % Hamamelis Virginiana Extract (CAS-n° 84696-19-5), 0,06000 % Hordeum Vulgare Cera (CAS-n° 85251-64-5), 0,06000 % Stevia Rebaudiana Extract (CAS-n° 91722-21-3), 0,03000 % Propylparaben (CAS-n° 94-13-3), 0,02000 % Citrus Grandis Oil (CAS-n° 8016-20-4), 0,01600 % Vanilla Planifolia Extract (CAS-n° 84650-63-5), 0,01500 % Elettaria Cardamon Oil (CAS-n° 8000-66-6), 0,00860 % Hexyl Cinnamal (CAS-n° 101-86-0), 0,00730 % Benzyl Benzoate (CAS-n° 120-51-4), 0,00600 % Linalool (CAS-n° 78-70-6), 0,00417 % Limonene, 0,00240 % Hyaluronic Acid (CAS-n° 9004-61-9), 0,00200 % alpha-Isomethyl lonone, 0,00239 % Tocopherol (CAS-n° 10191-41-0), 0,00180 % Citronellol (CAS-n° 106-22-9), 0,00056 % Retinyl Palmitate (CAS-n° 79-81-2) et de l'eau.

13. Préparation selon l'une des revendications 1 à 10, **caractérisée en ce que**
a) ladite préparation cosmétique se présente sous forme d'un tonique capillaire,
b) ladite préparation cosmétique, laquelle se présente sous forme d'un tonique capillaire, contenant le cas échéant, par rapport au poids total de ladite préparation, ≥ 50 % Aqua (CAS-n° 7732-18-5), 10 à 25 % Mipa-Laureth Sulfate (CAS-n° 83016-76-6/9062-04-8), 1 à 5 % Mipa-Lauryl Sulfate (CAS-n° 21142-28-9), 1 à 5 % Cocoamidopropyl Betain (CAS-n° 263-058-8), 1 à 5 % Disodium Laureth Sulfosuccinate (CAS-n° 39354-45-5), 1 à 5 % PEG-12 Glyceryl Laurate (CAS-n° 59070-56-3), 1 à 5 % Disodium Cocoamphodiacetate (CAS-n° 68650-39-5), 1 à 5 % Laureth-13 (CAS-n° 9002-92-0), 1 à 5 % Glycoproteins (CAS-n° 84604-16-0), 0,1 à 1% PEG-36 Castor Oil (CAS-n° 61791-12-6), 0,1 à 1 % Propylene Glycol (CAS-n° 57-55-6), 0,1 à 1 % Butylene Glycol (CAS-n° 107-88-0), 0,1 à 1 % Panthenol (CAS-n° 81-13-0), 0,1 à 1 % Linoleamide DEA (CAS-n° 56863-02-6), 0,1 à 1 % Farnesyl Acetate (CAS-n° 29548-30-9), 0,1 à 1 % Farnesol (CAS-n° 4602-84-0), 0,1 à 1 % Parfum, 0,1 à 1 % PEG-10 Olive Glycerides, 0,1 à 1 % Imidazolidinyl Urea (CAS-n° 39236-46-9), 0,1 à 1 % Hydrolyzed Milk Protein (CAS-n° 92797-39-2/8049-98-7), 0,1 à 1 % Olea Europaea Leaf Extract (CAS-n° 84012-27-1),0,1 à 1 % Centella Asiatica Extract (CAS-n° 84696-21-9), 0,1 à 1 % Aspalathus Linearis Extract (CAS-n° 776295-36-4), 0,1 à 1 % Sales, 0,1 à 1 % Nelumbo Nucifera Extract (CAS-n° 85085-51-4), 0,1 à 1 % Hamamelis Virginiana Extract (CAS-n° 84696-19-5), 0,1 à 1 % Methylparaben (CAS-n° 99-76-3), 0,1 à 1 % Panicum Miliaceum Extract (CAS-n° 90082-36-3), 0,1 à 1 % Salvia Officinalis Extract (CAS-n° 84082-79-1), 0,1 à 1 % Adenosine Triphosphate (CAS-n° 56-65-5), 0,1 à 1 % Betula Alba Leaf Extract (CAS-n° 84012-15-7), 0,1 à 1 % Polyquaternium-10 (CAS-n° 81859-24-7/53568-66-4), 0,1 à 1 % Chamomilla Recutita Extract (CAS-n° 84082-60-0), 0,1 à 1 % Niacinamide (CAS-n° 98-92-0), < 0,1 % Camelia Sinensis Extract (CAS-n° 84650-60-2), < 0,1 % Tilia Cordata Flower Extract (CAS-n° 84929-52-2), < 0,1 % Urtica Dioica Extract (CAS-n° 84012-40-8), < 0,1 % Panthenyl Triacetate (CAS-n° 94089-18-6), < 0,1 % Humulus Lupulus Extract (CAS-n° 8060-28-4), < 0,1 % Cinchona Pubescens Extract (CAS-n° 84776-28-3), < 0,1 % Equisetum Arvense Extract (CAS-n° 71011-23-9), < 0,1 % Stevia Rebaudiana Extract (CAS-n° 91722-21-3), < 0,1 % Citrus Grandis Oil (CAS-n° 8016-20-4), < 0,1 % Vanilla Planifolia Extract (CAS-n° 84650-63-5), < 0,1 % Elettaria Cardamon Oil (CAS-n° 8000-66-6), < 0,1 % Propylparaben (CAS-n° 94-13-3), < 0,1 % Hexyl Cinnamal (CAS-n° 101-86-0), < 0,1 % Benzyl Benzoate (CAS-n° 120-51-4), < 0,1 % Linalool (CAS-n° 78-70-6), < 0,1 % Limonene, < 0,1 % Niacin (CAS-n° 59-67-6), < 0,1 % Hyaluronic Acid (CAS-n° 9004-61-9) et < 0,1 % Biotin (CAS-n° 58-85-5) et
c) ladite préparation cosmétique, laquelle se présente sous forme d'un tonique capillaire, contenant le cas échéant, par rapport au poids total de ladite préparation, 10,00000 % Mipa-Laureth Sulfate (CAS-n° 83016-76-6/9062-04-8), 3,15000 % Mipa-Lauryl Sulfate (CAS-n° 21142-28-9), 1,60000 % Cocoamidopropyl Betain (CAS-n° 263-058-8), 1,50000 % Disodium Laureth Sulfosuccinate (CAS-n° 39354-45-5), 1,20000 % PEG-12 Glyceryl Laurate (CAS-n° 59070-56-3), 1,20000 % Disodium Cocoamphodiacetate (CAS-n° 68650-39-5), 1,20000 % Laureth-13 (CAS-n° 9002-92-0), 1,00000 % Glycoproteins (CAS-n° 84604-16-0), 0,90000 % PEG-36 Castor Oil (CAS-n° 61791-12-6), 0,86000 % Propylene Glycol (CAS-n° 57-55-6), 0,60000 % Butylene Glycol (CAS-n° 107-88-0), 0,54990 % Panthenol (CAS-n° 81-13-0), 0,45000 % Linoleamide DEA (CAS-n° 56863-02-6), 0,45000 % Farnesyl Acetate (CAS-n° 29548-30-9), 0,36000 % Farnesol (CAS-n° 4602-84-0), 0,34927 % Parfum, 0,30000 % PEG-10 Olive Glycerides, 0,30000 % Imidazolidinyl Urea (CAS-n° 39236-46-9), 0,20000 % Hydrolyzed Milk Protein (CAS-n° 92797-39-2/8049-98-7), 0,15000 % Olea Europaea Leaf Extract (CAS-n° 84012-27-1), 0,15000 % Centella Asiatica Extract (CAS-n° 84696-21-9), 0,15000 % Aspalathus Linearis Extract (CAS-n° 776295-36-4), 0,15000 % Sales, 0,11250 % Nelumbo Nucifera Extract (CAS-n° 85085-51-4), 0,11250 % Hamamelis Virginiana Extract (CAS-n° 84696-19-5), 0,11000 % Methylparaben (CAS-n° 99-76-3), 0,10000 % Panicum Miliaceum Extract (CAS-n° 90082-36-3), 0,10000 % Salvia Officinalis Extract (CAS-n° 84082-79-1), 0,10000 % Adenosine Triphosphate (CAS-n° 56-65-5), 0,10000 % Betula Alba Leaf Extract (CAS-n° 84012-15-7), 0,10000 % Polyquaternium-10 (CAS-n° 81859-24-7/53568-66-4), 0,10000 % Chamomilla Recutita Extract (CAS-n° 84082-60-0), 0,10000 % Niacinamide (CAS-n° 98-92-0), 0,09000 % Camelia Sinensis Extract (CAS-n° 84650-60-2), 0,09000 % Tilia Cordata Flower Extract (CAS-n° 84929-52-2), 0,09000 % Urtica Dioica Extract (CAS-n° 84012-40-8), 0,09000 % Panthenyl Triacetate (CAS-n° 94089-18-6), 0,09000 % Humulus Lupulus Extract (CAS-n° 8060-28-4), 0,09000 % Cinchona Pubescens Extract (CAS-n° 84776-28-3), 0,09000 % Equisetum Arvense Extract (CAS-n° 71011-23-9), 0,07500 % Stevia Rebaudiana Extract (CAS-n° 91722-21-3), 0,06000 % Citrus Grandis Oil (CAS-n° 8016-20-4), 0,04800 % Vanilla Planifolia Extract (CAS-n° 84650-63-5), 0,04500 % Elettaria Cardamon Oil (CAS-n° 8000-66-6), 0,03000 % Propylparaben (CAS-n° 94-13-3), 0,02580 % Hexyl Cinnamal (CAS-n° 101-86-0), 0,02190 % Benzyl Benzoate (CAS-n° 120-51-4), 0,01801 % Linalool (CAS-n° 78-70-6), 0,01252 % Limonene, 0,01000 % Niacin (CAS-n° 59-67-6), 0,00300 % Hyaluronic Acid (CAS-n° 9004-61-9), 0,00010 % Biotin (CAS-n° 58-85-5) et de l'eau.

14. Préparation selon l'une des revendications 1 à 10, **caractérisée en ce que**
a) ladite préparation cosmétique se présente sous forme d'un shampooing-douche,
b) ladite préparation cosmétique, laquelle se présente sous forme d'un shampooing-douche, contenant le cas échéant, par rapport au poids total de ladite préparation, ≥ 50 % Aqua (CAS-n° 7732-18-5), 10 à 25 % Mipa-Laureth Sulfate (CAS-n° 83016-76-6/9062-04-8), 1 à 5 % Mipa-Lauryl Sulfate (CAS-n° 21142-28-9), 1 à 5 % Cocoamidopropyl Betain (CAS-n° 263-058-8), 1 à 5 % Disodium Laureth Sulfosuccinate (CAS-n° 39354-45-5), 1 à 5 % Laureth-13 (CAS-n° 9002-92-0), 1 à 5 % Disodium Cocoamphodiacetate (CAS-n° 68650-39-5), 0,1 à 1 % Sodium Chloride (CAS-n° 7647-14-5), 0,1 à 1 % Propylene Glycol (CAS-n° 57-55-6), 0,1 à 1 % Glycoproteins (CAS-n° 84604-16-0), 0,1 à 1 % Linoleamide DEA (CAS-n° 56863-02-6), 0,1 à 1 % Panthenol (CAS-n° 81-13-0), 0,1 à 1 % Polyquaternium-10 (CAS-n° 81859-24-7/53568-66-4), 0,1 à 1 % PEG-10 Olive Glycerides, 0,1 à 1 % Imidazolidinyl Urea (CAS-n° 39236-46-9), 0,1 à 1 % Panicum Miliaceum Extract (CAS-n° 90082-36-3), 0,1 à 1 % Chamomilla Recutita Extract (CAS-n° 84082-60-0), 0,1 à 1 % Salvia Officinalis Extract (CAS-n° 84082-79-1), 0,1 à 1 % Betula Alba Leaf Extract (CAS-n° 84012-15-7), 0,1 à 1 % Parfum, 0,1 à 1 % Tilia cordata Flower Extract (CAS-n° 84929-52-2), 0,1 à 1 % Urtica Dioica Extract (CAS-n° 84012-40-8), 0,1 à 1 % Camelia Sinensis Extract (CAS-n° 84650-60-2), 0,1 à 1 % Equisetum Arvense Extract (CAS-n° 71011-23-9), 0,1 à 1 % Humulus Lupulus Extract (CAS-n° 8060-28-4), 0,1 à 1 % Cinchona Extract (CAS-n° 84776-28-3), 0,1 à 1 % Sales, 0,1 à 1 % PEG-12 Glyceryl Laurate (CAS-n° 59070-56-3), 0,1 à 1 % Methylparaben (CAS-n° 99-76-3), < 0,1 % PEG-36 Castor Oil (CAS-n° 61791-12-6), < 0,1 % Centella Asiatica Extract (CAS-n° 84696-21-9), < 0,1 % Olea Europaea Leaf Extract (CAS-n° 84012-27-1), < 0,1 % Aspalathus Linearis Extract (CAS-n° 776295-36-4), < 0,1 % Hamamelis Virginiana Extract (CAS-n° 84696-19-5), < 0,1 % Nelumbo Nucifera Extract (CAS-n° 85085-51-4), < 0,1 % Farnesyl Acetate (CAS-n° 29548-30-9), < 0,1 % Citrus Grandis Oil (CAS-n° 8016-20-4), < 0,1 % Stevia Rebaudiana Extract (CAS-n° 91722-21-3), < 0,1 % Farnesol (CAS-n° 4602-84-0), < 0,1 % Vanilla Planifolia Extract (CAS-n° 84650-63-5), < 0,1 % Elettaria Cardamon Oil (CAS-n° 8000-66-6), < 0,1 % Propylparaben (CAS-n° 94-13-3), < 0,1 % Niacin (CAS-n° 59-67-6), < 0,1 % Hexyl Cinnamal (CAS-n° 101-86-0), < 0,1 % Benzyl Benzoate (CAS-n° 120-51-4), < 0,1 % Linalool (CAS-n° 78-70-6) et < 0,1% Hyaluronic Acid (CAS-n° 9004-61-9) et
c) ladite préparation cosmétique, laquelle se présente sous forme d'un shampooing-douche, contenant le cas échéant, par rapport au poids total de ladite préparation, 10,00000 % Mipa-Laureth Sulfate (CAS-n° 83016-76-6/9062-04-8), 3,15000 % Mipa-Lauryl Sulfate (CAS-n° 21142-28-9), 1,60000 % Cocoamidopropyl Betain (CAS-n° 263-058-8), 1,50000 % Disodium Laureth Sulfosuccinate (CAS-n° 39354-45-5), 1,20000 % Laureth-13 (CAS-n° 9002-92-0), 1,20000 % Disodium Cocoamphodiacetate (CAS-n° 68650-39-5), 0,90000 % Sodium Chloride (CAS-n° 7647-14-5), 0,71000 % Propylene Glycol (CAS-n° 57-55-6), 0,50000 % Glycoproteins (CAS-n° 84604-16-0), 0,45000 % Linoleamide DEA (CAS-n° 56863-02-6), 0,37475 % Panthenol (CAS-n° 81-13-0), 0,30000 % Polyquaternium-10 (CAS-n° 81859-24-7/53568-66-4), 0,30000 % PEG-10 Olive Glycerides, 0,30000 % Imidazolidinyl Urea (CAS-n° 39236-46-9), 0,25000 % Panicum Miliaceum Extract (CAS-n° 90082-36-3), 0,25000 % Chamomilla Recutita Extract (CAS-n° 84082-60-0), 0,25000 % Salvia Officinalis Extract (CAS-n° 84082-79-1), 0,25000 % Betula Alba Leaf Extract (CAS-n° 84012-15-7), 0,23285 % Parfum, 0,22500% Tilia Cordata Flower Extract (CAS-n° 84929-52-2), 0,22500 % Urtica Dioica Extract (CAS-n° 84012-40-8), 0,22500 % Camelia Sinensis Extract (CAS-n° 84650-60-2), 0,22500 % Equisetum Arvense Extract (CAS-n° 71011-23-9), 0,22500 % Humulus Lupulus Extract (CAS-n° 8060-28-4), 0,22500 % Cinchona Extract (CAS-n° 84776-28-3), 0,15000 % Sales, 0,12000 % PEG-12 Glyceryl Laurate (CAS-n° 59070-56-3), 0,11000 % Methylparaben (CAS-n° 99-76-3), 0,09000 % PEG-36 Castor Oil (CAS-n° 61791-12-6), 0,075001 % Centella Asiatica Extract (CAS-n° 84696-21-9), 0,07500 % Olea Europaea Leaf Extract (CAS-n° 84012-27-1), 0,07500 % Aspalathus Linearis Extract (CAS-n° 776295-36-4), 0,05625 % Hamamelis Virginiana Extract (CAS-n° 84696-19-5), 0,05625 % Nelumbo Nucifera Extract (CAS-n° 85085-51-4), 0,04500 % Farnesyl Acetate (CAS-n° 29548-30-9), 0,04000 % Citrus Grandis Oil (CAS-n° 8016-20-4), 0,03750 % Stevia Rebaudiana Extract (CAS-n° 91722-21-3), 0,03600 % Farnesol (CAS-n° 4602-84-0), 0,03200 % Vanilla Planifolia Extract (CAS-n° 84650-63-5), 0,03000 % Elettaria Cardamon Oil (CAS-n° 8000-66-6), 0,03000 % Propylparaben (CAS-n° 94-13-3), 0,02500 % Niacin (CAS-n° 59-67-6), 0,01720 % Hexyl Cinnamal (CAS-n° 101-86-0), 0,01460 % Benzyl Benzoate (CAS-n° 120-51-4), 0,01201 % Linalool (CAS-n° 78-70-6), 0,00150 % Hyaluronic Acid (CAS-n° 9004-61-9) et de l'eau.

15. Utilisation d'une eau thermale de type sodium - chlorure - fer - iode ayant une teneur globale en électrolytes supérieure ou égale à 25 g/kg, de préférence supérieure ou égale à 30 g/kg, et une teneur en fer supérieure ou égale à 9 mg/kg, pour fabriquer une préparation cosmétique destinée aux soins de la peau selon l'une des revendications 1 à 14.
